# EUROPEAN PATENT APPLICATION

(11) **EP 4 428 205 A1**
(43) Date of publication of application: **11.09.2024**
(21) Application number: 22889985.2
(22) Date of filing: 02.11.2022
(51) Int. Cl.: C09D 5/16, C07K 16/00, C08F 220/28, C08F 220/34, C08F 230/02, C09D 133/04, C09D 133/14, C09D 143/02, C09D 157/06, C09D 7/65

(54) **ADHESION INHIBITOR FOR BIOLOGICAL SUBSTANCES**

(30) Priority: 02.11.2021 JP 2021179721; 02.11.2021 JP 2021179722
(71) Applicant: Nissan Chemical Corporation, Tokyo 103-6119 (JP)
(72) Inventor: UEDA, Yuki, Funabashi-shi, Chiba 274-0052 (JP); HIROI, Yoshiomi, Funabashi-shi, Chiba 274-0052 (JP); SUZUKI, Kohei, Funabashi-shi, Chiba 274-0052 (JP); HIROI, Miya, Funabashi-shi, Chiba 274-0052 (JP); NAKAJIMA, Hiroyuki, Shiraoka-shi, Saitama 349-0294 (JP); SASATSUKI, Hitoshi, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2022/041017
(87) International publication number: WO 2023/080165

(57) **Abstract**

This is to provide a coating film well balanced in desired characteristics.

The present invention is to provide a composition for forming a coating film which comprises (1) a polymer of a monomer mixture containing monomers represented by the following formulae (A) to (D): 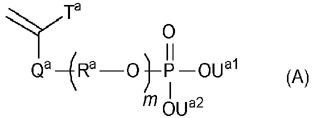 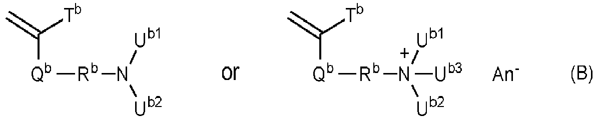 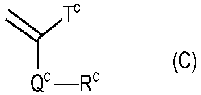 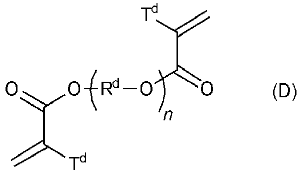 (wherein T^{a}, T^{b}, T^{c}, T^{d}, U^{a1}, U^{a2}, U^{b1}, U^{b2}, U^{b3}, Q^{a}, Q^{b}, Q^{c}, R^{a}, R^{b}, R^{c}, R^{d}, An⁻, m and n are as defined in the specification and Claims), where a ratio of a total of the anionic monomer represented by the above-mentioned formula (A) and the cationic monomer represented by the above-mentioned formula (B) based on total monomers contained in the monomer mixture is 40 mol% or more, and (2) a polycarbodiimide containing a structure represented by the following formula (E):

-N=C=N- (E)

a coating film which is a cured product thereof and a process for producing the same, and a cured product and a process for producing the same.

## Description

### TECHNICAL FIELD

The present invention relates to a composition for forming a coating film, a coating film which is a cured product thereof and a process for producing the same, and a cured product and a process for producing the same.

### BACKGROUND ART

In order to suppress adhesion of biological substances such as cells, proteins, sugars, etc., coating materials capable of suppressing adhesion of various biological substances have been proposed that can be applied to medical or research equipment and instruments, etc. In addition, in recent years, research and development or marketing of antibody drugs have progressed, and the demand for coating materials capable of suppressing adhesion of biological substances that can be applied to their storage containers (product containers) is increasing.

The present inventors have previously reported that a coating agent containing a copolymer that contains a specific anionic group and cationic group can be firmly adhered to any type of substrate, and after adhesion, it becomes a coating film excellent in resistance to aqueous solvents, and shows an excellent ability to suppress adhesion of biological substances (for example, see Patent Documents 1 and 2).

### PRIOR ART DOCUMENTS

### PATENT DOCUMENTS

| | |
|---|---|
| Patent Document 1: | WO 2014/196650 |
| Patent Document 2: | WO 2021/167037 |

### SUMMARY OF THE INVENTION

### PROBLEMS TO BE SOLVED BY THE INVENTION

The present inventors reported that a coating agent containing a copolymer which contains a specific anionic group and cationic group forms an ion complex, is capable of forming a coating film having an excellent ability to suppress adhesion of biological substances, and that the coating film has excellent in adhesion to the base material. However, for example, in the coating film for storage containers of antibody drug, further improvement is required in terms of suppression of aggregation of antibody and dissolution of the components of the coating film into the drugs, as well as in the ability of suppressing adhesion of biological substances.

The present inventors found that by further curing an ion complex material containing a specific bifunctional monomer that can be a crosslinking component with a specific crosslinking agent while controlling a ratio of the ion complex, a coating film with a high ability of suppressing aggregation of antibody as well as a high ability to suppress adhesion of biological materials as before, and with improved suppression of dissolution, and well balanced in desired characteristics, the present invention was completed.

### MEANS TO SOLVE THE PROBLEMS

The present invention is as follows.
[1] A composition for forming a coating film which comprises
   (1) a polymer of a monomer mixture which contains an anionic monomer represented by the following formula (A): 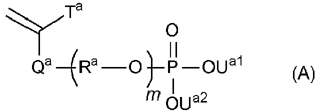 (wherein
      T^{a}, U^{a1} and U^{a2} each independently represent a hydrogen atom or a linear or branched alkyl group having 1 to 5 carbon atoms;
      Q^{a} represents a single bond, an ester bond or an amide bond;
      R^{a} represents a linear or branched alkylene group having 1 to 10 carbon atoms which may be substituted by a halogen atom(s); and
      m represents an integer of 1 to 10),
      a cationic monomer represented by the following formula (B): 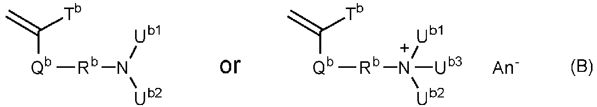 (wherein
         T^{b}, U^{b1}, U^{b2} and U^{b3} each independently represent a hydrogen atom or a linear or branched alkyl group having 1 to 5 carbon atoms;
         Q^{b} represents a single bond, an ester bond or an amide bond;
         R^{b} represents a linear or branched alkylene group having 1 to 10 carbon atoms which may be substituted by a halogen atom(s); and
         An⁻ represents an anion selected from the group consisting of a halide ion, an inorganic acid ion, a hydroxide ion and an isothiocyanate ion),
         a hydrophobic monomer represented by the following formula (C): 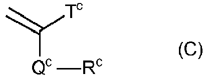 [wherein
            T^{c} represents a hydrogen atom or a linear or branched alkyl group having 1 to 5 carbon atoms;
            Q^{c} represents a single bond, an ether bond or an ester bond;
            R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)],
            and a bifunctional monomer represented by the following formula (D): 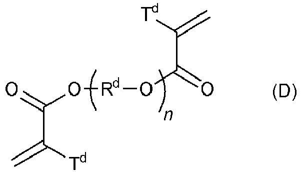 (wherein
               T^{d} represents a hydrogen atom or a linear or branched alkyl group having 1 to 6 carbon atoms;
               R^{d} represents a linear or branched alkylene group having 1 to 10 carbon atoms which may be substituted by a halogen atom(s); and
               n represents an integer of 1 to 10),
               where a ratio of a total of the anionic monomer represented by the above-mentioned formula (A) and the cationic monomer represented by the above-mentioned formula (B) based on total monomers contained in the monomer mixture is 40 mol% or more, and
   (2) a polycarbodiimide containing a structure represented by the following formula (E):

      -N=C=N- (E)
[2] The composition for forming a coating film described in [1], wherein a ratio of the bifunctional monomer represented by the above-mentioned formula (D) based on the total monomers contained in the above-mentioned monomer mixture is less than 30 mol%.
[3] The composition for forming a coating film described in [1] or [2], wherein the above-mentioned polycarbodiimide contains a hydrophilic group(s).
[4] The composition for forming a coating film described in [3], wherein the above-mentioned hydrophilic group is represented by the following formula (F):

   R¹-(O-CHR²-CH₂)ₒ- (F)

   (wherein
   R¹ represents a linear or branched alkyl group having 1 to 5 carbon atoms,
   R² represents a hydrogen atom or a methyl group, and when a plural number of R² are present, said R²s may be the same or different from each other, and o represents an integer of 1 to 30).
[5] A coating film which is a cured product of an applied film of the composition for forming a coating film described in any one of [1] to [4].
[6] The coating film described in [5], which has an ability to suppress adhesion of biological substances.
[7] A process for producing a coating film, which comprises a step of applying the composition for forming a coating film described in any one of [1] to [4] onto a substrate to form an applied film, and a step of drying the above-mentioned applied film to form a cured product.
[8] The process for producing a coating film described in [7], which further comprises a step of washing the cured product obtained after the drying step with a water-containing alcohol solvent.
[9] A cured product of a polymer of a monomer mixture which comprises an anionic monomer represented by the following formula (A): 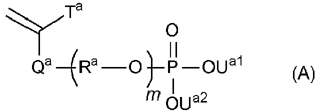 (wherein
   T^{a}, U^{a1} and U^{a2} each independently represent a hydrogen atom or a linear or branched alkyl group having 1 to 5 carbon atoms;
   Q^{a} represents a single bond, an ester bond or an amide bond;
   R^{a} represents a linear or branched alkylene group having 1 to 10 carbon atoms which may be substituted by a halogen atom(s); and
   m represents an integer of 1 to 10),
   a cationic monomer represented by the following formula (B): 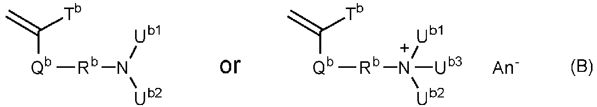 (wherein
      T^{b}, U^{b1}, U^{b2} and U^{b3} each independently represent a hydrogen atom or a linear or branched alkyl group having 1 to 5 carbon atoms;
      Q^{b} represents a single bond, an ester bond or an amide bond;
      R^{b} represents a linear or branched alkylene group having 1 to 10 carbon atoms which may be substituted by a halogen atom(s); and
      An⁻ represents an anion selected from the group consisting of a halide ion, an inorganic acid ion, a hydroxide ion and an isothiocyanate ion),
      a hydrophobic monomer represented by the following formula (C): 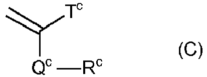 [wherein
         T^{c} represents a hydrogen atom or a linear or branched alkyl group having 1 to 5 carbon atoms;
         Q^{c} represents a single bond, an ether bond or an ester bond;
         R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)],
         and a bifunctional monomer represented by the following formula (D): 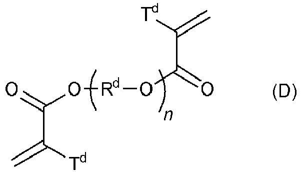 (wherein
            T^{d} represents a hydrogen atom or a linear or branched alkyl group having 1 to 6 carbon atoms;
            R^{d} represents a linear or branched alkylene group having 1 to 10 carbon atoms which may be substituted by a halogen atom(s); and
            n represents an integer of 1 to 10),
            where a ratio of a total of the anionic monomer represented by the above-mentioned formula (A) and the cationic monomer represented by the above-mentioned formula (B) based on total monomers contained in the monomer mixture is 40 mol% or more.
[10] The cured product described in [9], which contains a pyrophosphate structure.
[11] A process for preparing a cured product which comprises
   (i) a step of polymerizing a monomer mixture containing an anionic monomer represented by the following formula (A): 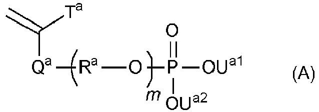 (wherein
      T^{a}, U^{a1} and U^{a2} each independently represent a hydrogen atom or a linear or branched alkyl group having 1 to 5 carbon atoms;
      Q^{a} represents a single bond, an ester bond or an amide bond;
      R^{a} represents a linear or branched alkylene group having 1 to 10 carbon atoms which may be substituted by a halogen atom(s); and
      m represents an integer of 1 to 10),
      a cationic monomer represented by the following formula (B): 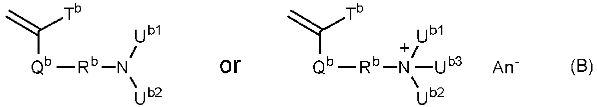 (wherein
         T^{b}, U^{b1}, U^{b2} and U^{b3} each independently represent a hydrogen atom or a linear or branched alkyl group having 1 to 5 carbon atoms;
         Q^{b} represents a single bond, an ester bond or an amide bond;
         R^{b} represents a linear or branched alkylene group having 1 to 10 carbon atoms which may be substituted by a halogen atom(s); and
         An⁻ represents an anion selected from the group consisting of a halide ion, an inorganic acid ion, a hydroxide ion and an isothiocyanate ion),
         a hydrophobic monomer represented by the following formula (C): 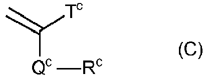 [wherein
            T^{c} represents a hydrogen atom or a linear or branched alkyl group having 1 to 5 carbon atoms;
            Q^{c} represents a single bond, an ether bond or an ester bond;
            R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)],
            and a bifunctional monomer represented by the following formula (D): 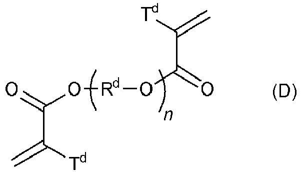 (wherein
               T^{d} represents a hydrogen atom or a linear or branched alkyl group having 1 to 6 carbon atoms;
               R^{d} represents a linear or branched alkylene group having 1 to 10 carbon atoms which may be substituted by a halogen atom(s); and
               n represents an integer of 1 to 10),
               where a ratio of a total of the anionic monomer represented by the above-mentioned formula (A) and the cationic monomer represented by the above-mentioned formula (B) based on total monomers contained in the monomer mixture is 40 mol% or more to obtain a copolymer, and
   (ii) a step of reacting the above-mentioned copolymer with a polycarbodiimide containing the structure represented by the following formula (E):

      -N=C=N- (E)
   to obtain a cured product.
[12] The coating film described in [5], which has an ability to suppress aggregation of antibody.
[13] A storage container of an antibody drug, which comprises having the coating film described in [12] onto at least part of the surface thereof.
[14] A composition for forming a coating film which comprises
   (1) a polymer of a monomer mixture which contains an anionic monomer represented by the following formula (A): 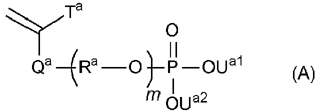 (wherein
      T^{a}, U^{a1} and U^{a2} each independently represent a hydrogen atom or a linear or branched alkyl group having 1 to 5 carbon atoms;
      Q^{a} represents a single bond, an ester bond or an amide bond;
      R^{a} represents a linear or branched alkylene group having 1 to 10 carbon atoms which may be substituted by a halogen atom(s); and
      m represents an integer of 1 to 10),
      a cationic monomer represented by the following formula (B): 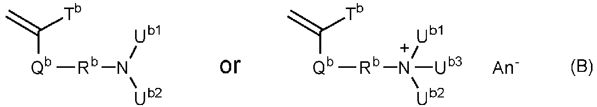 (wherein
         T^{b}, U^{b1}, U^{b2} and U^{b3} each independently represent a hydrogen atom or a linear or branched alkyl group having 1 to 5 carbon atoms;
         Q^{b} represents a single bond, an ester bond or an amide bond;
         R^{b} represents a linear or branched alkylene group having 1 to 10 carbon atoms which may be substituted by a halogen atom(s); and
         An⁻ represents an anion selected from the group consisting of a halide ion, an inorganic acid ion, a hydroxide ion and an isothiocyanate ion),
         and a hydrophobic monomer represented by the following formula (C): 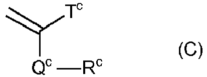 [wherein
            T^{c} represents a hydrogen atom or a linear or branched alkyl group having 1 to 5 carbon atoms;
            Q^{c} represents a single bond, an ether bond or an ester bond;
            R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)],
            where a ratio of a total of the above-mentioned anionic monomer represented by the formula (A) and the cationic monomer represented by the above-mentioned formula (B) based on a total monomers contained in the above-mentioned monomer mixture is 40 mol% or more, and
   (2) a polycarbodiimide containing a structure represented by the following formula (E):

      -N=C=N- (E)
[15] A coating film which is a cured product of an applied film of the composition for forming a coating film described in [14].
[16] The coating film described in [15], which has an ability to suppress aggregation of antibody.
[17] A storage container of an antibody drug which comprises having the coating film described in [15] or [16] onto at least part of the surface thereof.
[18] A composition for forming a coating film which comprises a polymer of a monomer mixture containing
   an anionic monomer represented by the following formula (A): 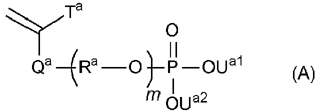 (wherein
   T^{a}, U^{a1} and U^{a2} each independently represent a hydrogen atom or a linear or branched alkyl group having 1 to 5 carbon atoms;
   Q^{a} represents a single bond, an ester bond or an amide bond;
   R^{a} represents a linear or branched alkylene group having 1 to 10 carbon atoms which may be substituted by a halogen atom(s); and
   m represents an integer of 1 to 10),
   a cationic monomer represented by the following formula (B): 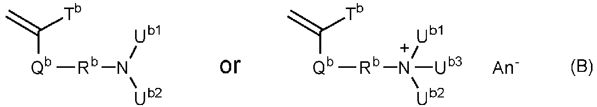 (wherein
      T^{b}, U^{b1}, U^{b2} and U^{b3} each independently represent a hydrogen atom or a linear or branched alkyl group having 1 to 5 carbon atoms;
      Q^{b} represents a single bond, an ester bond or an amide bond;
      R^{b} represents a linear or branched alkylene group having 1 to 10 carbon atoms which may be substituted by a halogen atom(s); and
      An⁻ represents an anion selected from the group consisting of a halide ion, an inorganic acid ion, a hydroxide ion and an isothiocyanate ion),
      a hydrophobic monomer represented by the following formula (C): 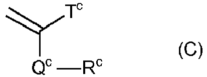 [wherein
         T^{c} represents a hydrogen atom or a linear or branched alkyl group having 1 to 5 carbon atoms;
         Q^{c} represents a single bond, an ether bond or an ester bond;
         R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)],
         and a bifunctional monomer represented by the following formula (D): 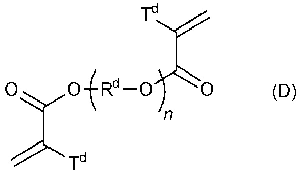 (wherein
            T^{d} represents a hydrogen atom or a linear or branched alkyl group having 1 to 6 carbon atoms;
            R^{d} represents a linear or branched alkylene group having 1 to 10 carbon atoms which may be substituted by a halogen atom(s); and
            n represents an integer of 1 to 10),
            where a ratio of a total of the above-mentioned anionic monomer represented by the formula (A) and the cationic monomer represented by the above-mentioned formula (B) based on the total monomers contained in the above-mentioned monomer mixture is 40 mol% or more, and having an ability to suppress aggregation of antibody.
[19] A coating film having an ability to suppress aggregation of antibody, which comprises a polymer of a monomer mixture containing
   an anionic monomer represented by the following formula (A): 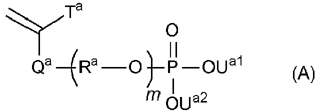 (wherein
   T^{a}, U^{a1} and U^{a2} each independently represent a hydrogen atom or a linear or branched alkyl group having 1 to 5 carbon atoms;
   Q^{a} represents a single bond, an ester bond or an amide bond;
   R^{a} represents a linear or branched alkylene group having 1 to 10 carbon atoms which may be substituted by a halogen atom(s); and
   m represents an integer of 1 to 10),
   a cationic monomer represented by the following formula (B): 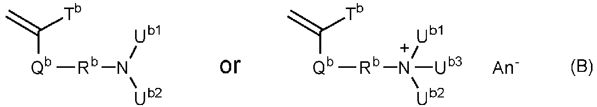 (wherein
      T^{b}, U^{b1}, U^{b2} and U^{b3} each independently represent a hydrogen atom or a linear or branched alkyl group having 1 to 5 carbon atoms;
      Q^{b} represents a single bond, an ester bond or an amide bond;
      R^{b} represents a linear or branched alkylene group having 1 to 10 carbon atoms which may be substituted by a halogen atom(s); and
      An⁻ represents an anion selected from the group consisting of a halide ion, an inorganic acid ion, a hydroxide ion and an isothiocyanate ion),
      a hydrophobic monomer represented by the following formula (C): 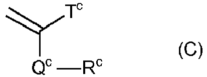 [wherein
         T^{c} represents a hydrogen atom or a linear or branched alkyl group having 1 to 5 carbon atoms;
         Q^{c} represents a single bond, an ether bond or an ester bond;
         R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)],
         and a bifunctional monomer represented by the following formula (D): 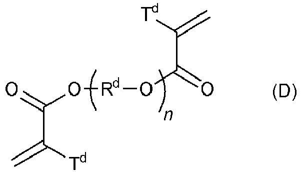 (wherein
            T^{d} represents a hydrogen atom or a linear or branched alkyl group having 1 to 6 carbon atoms;
            R^{d} represents a linear or branched alkylene group having 1 to 10 carbon atoms which may be substituted by a halogen atom(s); and
            n represents an integer of 1 to 10),
            where a ratio of a total of the above-mentioned anionic monomer represented by the formula (A) and the cationic monomer represented by the above-mentioned formula (B) based on the total monomers contained in the above-mentioned monomer mixture is 40 mol% or more, and having an ability to suppress aggregation of antibody.
[20] A storage container of an antibody drug which comprises having the coating film described in [19] onto at least part of the surface thereof.

### EFFECTS OF THE INVENTION

The coating film of the present invention can be formed by drying a composition for forming a coating film which comprises a polymer of a monomer mixture containing an anionic monomer represented by the formula (A), a cationic monomer represented by the formula (B), a hydrophobic monomer represented by the formula (C), and a bifunctional monomer represented by the formula (D), wherein amounts of the anionic monomer represented by the formula (A) and the above-mentioned cationic monomer represented by the formula (B) is in a specific ratio, and a specific crosslinking agent, after producing a film. That is, in the coating film of the present invention, in addition to crosslinking by a bifunctional monomer, by proceeding curing with post-crosslinking by a specific crosslinking agent while controlling a ratio of the ion complex, further improvements are made, in addition to an ability to suppress adhesion of biological substances as before, in the points of suppressing aggregation of antibody or dissolution of the coating film components into the drugs, whereby it becomes a coating film well balanced in desired characteristics.

### EMBODIMENTS OF THE INVENTION

### «Explanation of terms»

The terms used in the present invention have the following definitions unless otherwise specifically mentioned.

In the present invention, the "halogen atom" means a fluorine atom, a chlorine atom, a bromine atom or an iodine atom.

In the present invention, the "alkyl group" means a monovalent group of a linear or branched saturated aliphatic hydrocarbon. As the "linear or branched alkyl group having 1 to 5 carbon atoms," there may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group. As the "linear or branched alkyl group having 1 to 6 carbon atoms," there may be mentioned, in addition to the examples of the "linear or branched alkyl group having 1 to 5 carbon atoms," a hexyl group or an isomer thereof. Similarly, as the "linear or branched alkyl group having 1 to 18 carbon atoms," in addition to the examples of the "linear or branched alkyl group having 1 to 5 carbon atoms," there may be mentioned a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group or an octadecyl group, or an isomer thereof.

In the present invention, the "linear or branched alkyl group having 1 to 5 carbon atoms which may be substituted by a halogen atom" means the above-mentioned linear or branched alkyl group having 1 to 5 carbon atoms, or the above-mentioned linear or branched alkyl group having 1 to 5 carbon atoms substituted by one or more above-mentioned halogen atom(s). Examples of the "linear or branched alkyl group having 1 to 5 carbon atoms" are as mentioned above. On the other hand, the "linear or branched alkyl group having 1 to 5 carbon atoms" means ones in which one or more optional hydrogen atoms of the above-mentioned linear or branched alkyl group having 1 to 5 carbon atoms is/are substituted by a halogen atom(s), and examples may be mentioned a fluoromethyl group, a difluoromethyl group, a trifluoromethyl group, a chloromethyl group, a dichloromethyl group, a trichloromethyl group, a bromomethyl group, an iodomethyl group, a 2,2,2-trifluoroethyl group, a 2,2,2-trichloroethyl group, a perfluoroethyl group, a perfluorobutyl group or a perfluoropentyl group, etc.

In the present invention, the "ester bond" means -C(=O)-O- or -O-C(=O)-, the "amide bond" means -NHC(=O)- or -C(=O)NH-, and the ether bond means -O-.

In the present invention, the "linear or branched alkylene group having 1 to 10 carbon atoms which may be substituted by a halogen atom(s)" means a linear or branched alkylene group having 1 to 10 carbon atoms, or a linear or branched alkylene group having 1 to 10 carbon atoms substituted by one or more halogen atom(s). Here, the "alkylene group" means a divalent organic group corresponding to the above-mentioned alkyl group. Examples of the "linear or branched alkylene group having 1 to 10 carbon atoms" may be mentioned a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, a hexamethylene group, an octamethylene group and a decamethylene group, etc., and among these, an ethylene group, a propylene group, an octamethylene group and a decamethylene group are preferable, for example, more preferably a linear or branched alkylene group having 1 to 5 carbon atoms such as an ethylene group, a propylene group, a trimethylene group, a tetramethylene group, etc., and particularly preferably an ethylene group or a propylene group. The "linear or branched alkylene group having 1 to 10 carbon atoms substituted by one or more halogen atom(s) means ones in which one or more optional hydrogen atom(s) of the above-mentioned alkylene group is/are substituted by a halogen atom(s), and particularly preferably ones in which a part or whole of the hydrogen atom(s) of the ethylene group or the propylene group is/are substituted by a halogen atom(s).

In the present invention, the "cyclic hydrocarbon group having 3 to 10 carbon atoms" means a monocyclic or polycyclic, saturated or partially unsaturated, monovalent aliphatic hydrocarbon group having 3 to 10 carbon atoms. Among these, a monocyclic or bicyclic, saturated monovalent aliphatic hydrocarbon group having 3 to 10 carbon atoms are preferable, and for example, there may be mentioned a cycloalkyl group having 3 to 10 carbon atoms such as a cyclopropyl group, a cyclobutyl group or cyclohexyl group, etc., or a bicycloalkyl group having 4 to 10 carbon atoms such as a bicyclo[3.2. 1]octyl group, a bornyl group, an isobornyl group, etc.

In the present invention, the "aryl group having 6 to 10 carbon atoms" means a monocyclic or polycyclic, monovalent aromatic hydrocarbon group having 6 to 10 carbon atoms, and for example, there may be mentioned a phenyl group, a naphthyl group or an anthryl group, etc. The "aryl group having 6 to 10 carbon atoms" may be substituted by one or more above-mentioned "linear or branched alkyl group having 1 to 5 carbon atoms which may be substituted by a halogen atom(s)".

In the present invention, the "aralkyl group having 7 to 14 carbon atoms" means a group -R-R' (here, R represents the above-mentioned "alkylene group having 1 to 5 carbon atoms," and R' represents the above-mentioned "aryl group having 6 to 10 carbon atoms"), and for example, there may be mentioned a benzyl group, a phenethyl group or an α-methylbenzyl group, etc. The aryl portion of the "aralkyl group having 7 to 14 carbon atoms" may be substituted by one or more above-mentioned "linear or branched alkyl groups having 1 to 5 carbon atoms which may be substituted by a halogen atom(s)".

In the present invention, the "aryloxyalkyl group having 7 to 14 carbon atoms" means a group -R-O-R' (here, R represents the above-mentioned "alkylene group having 1 to 5 carbon atoms," and R' represents the above-mentioned "aryl group having 6 to 10 carbon atoms"), and for example, there may be mentioned a phenoxymethyl group, a phenoxyethyl group, or a phenoxypropyl group, etc. The aryl portion of the "aryloxyalkyl group having 7 to 14 carbon atoms" may be substituted by one or more above-mentioned "linear or branched alkyl groups having 1 to 5 carbon atoms which may be substituted by a halogen atom(s)".

In the present invention, the "halide ion" means a fluoride ion, a chloride ion, a bromide ion or an iodide ion.

In the present invention, the "inorganic acid ion" means a carbonate ion, a sulfate ion, a phosphate ion, a hydrogen phosphate ion, a dihydrogen phosphate ion, a nitrate ion, a perchlorate ion or a borate ion.

As the preferred An⁻ mentioned above, preferred are a halide ion, a sulfate ion, a phosphate ion, a hydroxide ion and an isothiocyanate ion, and particularly preferred is a halide ion.

In the present invention, the (meth)acrylate compound means both of an acrylate compound and a methacrylate compound. For example, (meth)acrylic acid means acrylic acid and methacrylic acid.

In the present invention, the "anionic monomer" means a monomer having an anionic group, and also include those having a group capable of being anionic by dissociating in water. Similarly, in the present invention, the "cationic monomer" means a monomer having a cationic group, and also include those having a group capable of being cationic by dissociating in water.

### «Explanation of the present invention»

### <Composition for forming coating film>

The first embodiment of the composition for forming a coating film of the present invention contains a polymer of a monomer mixture which contains (1) an anionic monomer represented by the formula (A), a cationic monomer represented by the formula (B), a hydrophobic monomer represented by the formula (C), and a bifunctional monomer represented by the formula (D), where a ratio of a total of the anionic monomer represented by the formula (A) and the cationic monomer represented by the formula (B) based on total monomers contained in the above-mentioned monomer mixture is 40 mol% or more, and (2) a polycarbodiimide containing a structure represented by the formula (E).

### (Polymer)

The monomer mixture according to the present invention contains an anionic monomer represented by the formula (A): 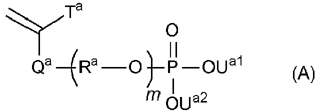 (wherein
T^{a}, U^{a1} and U^{a2} each independently represent a hydrogen atom or a linear or branched alkyl group having 1 to 5 carbon atoms;
Q^{a} represents a single bond, an ester bond or an amide bond;
R^{a} represents a linear or branched alkylene group having 1 to 10 carbon atoms which may be substituted by a halogen atom(s); and
m represents an integer of 1 to 10).
It may contain two or more kinds of the anionic monomers represented by the formula (A).

In one embodiment of the anionic monomer represented by the formula (A), as T^{a}, a hydrogen atom, a methyl group or an ethyl group is preferred, and a hydrogen atom or a methyl group is more preferred. As U^{a1} and U^{a2}, a hydrogen atom, a methyl group or an ethyl group is preferred, and a hydrogen atom is more preferred. As Q^{a}, a single bond or an ester bond is preferred, and an ester bond is more preferred. As R^{a}, a methylene group, an ethylene group or propylene group each of which may be substituted by a chlorine atom(s) is preferred, and an ethylene group or a propylene group is more preferred. m is preferably 2 to 8, and more preferably 3 to 6.

Specific examples of the monomer of the above-mentioned formula (A) may be mentioned acid phosphoxyethyl (meth)acrylate, 3-chloro-2-acid phosphoxypropyl (meth)acrylate, acid phosphoxypropyl (meth)acrylate, acid phosphoxymethyl (meth)acrylate, acid phosphoxypolyoxyethylene glycol mono(meth)acrylate and acid phosphoxypolyoxypropylene glycol mono(meth)acrylate, etc., and among these, acid phosphoxyethyl methacrylate (=phosphoric acid 2-(methacryloyloxy)ethyl), acid phosphoxypolyoxyethylene glycol monomethacrylate and acid phosphoxypolyoxypropylene glycol monomethacrylate are preferably used.

The structural formulae of acid phosphoxyethyl methacrylate (=phosphoric acid 2-(methacryloyloxy)ethyl), acid phosphoxypolyoxyethylene glycol monomethacrylate and acid phosphoxypolyoxypropylene glycol monomethacrylate are represented by the following formula (A-1) to formula (A-3), respectively. 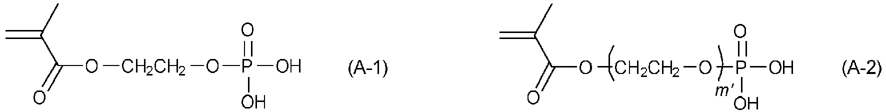 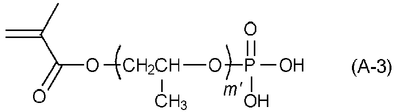 m'=3-6

The monomer mixture according to the present invention contains a cationic monomer represented by the formula (B): 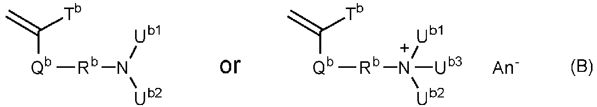 (wherein
T^{b}, U^{b1}, U^{b2} and U^{b3} each independently represent a hydrogen atom or a linear or branched alkyl group having 1 to 5 carbon atoms;
Q^{b} represents a single bond, an ester bond or an amide bond;
R^{b} represents a linear or branched alkylene group having 1 to 10 carbon atoms which may be substituted by a halogen atom(s); and
An⁻ represents an anion selected from the group consisting of a halide ion, an inorganic acid ion, a hydroxide ion and an isothiocyanate ion).
It may contain two or more kinds of the cationic monomers represented by the formula (B).

In one embodiment of the cationic monomer represented by the formula (B), as T^{b}, a hydrogen atom, a methyl group or an ethyl group is preferred, and a hydrogen atom or a methyl group is more preferred. As U^{b1}, U^{b2} and U^{b3}, each independently, a hydrogen atom, a methyl group, an ethyl group or a t-butyl group is preferred, and a methyl group or an ethyl group is more preferred. As Q^{b}, a single bond or an ester bond is preferred, an ester bond is more preferred. As R^{b}, a methylene group, an ethylene group or a propylene group each of which may be substituted by a chlorine atom(s) is preferred, and an ethylene group or a propylene group is more preferred. As An⁻, a halide ion is preferred, and a chloride ion is more preferred.

Specific examples of the above-mentioned monomer of the formula (B) may be mentioned dimethylaminoethyl (meth)acrylate, diethylaminoethyl (meth)acrylate, dimethylaminopropyl (meth)acrylate, 2-(t-butylamino)ethyl (meth)acrylate, methacryloylcholine chloride, etc., and among these, dimethylaminoethyl (meth)acrylate, methacryloylcholine chloride or 2-(t-butylamino)ethyl (meth)acrylate is preferably used.

The structural formulae of dimethylaminoethyl acrylate (=acrylic acid 2-(dimethylamino)ethyl), diethylaminoethyl methacrylate (=methacrylic acid 2-(diethylamino)ethyl), dimethylaminoethyl methacrylate (=methacrylic acid 2-(dimethylamino)ethyl), methacryloylcholine chloride and 2-(t-butylamino)ethyl methacrylate (=methacrylic acid 2-(t-butylamino)ethyl) are represented by the following formula (B-1) to formula (B-5), respectively.

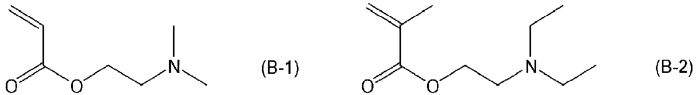

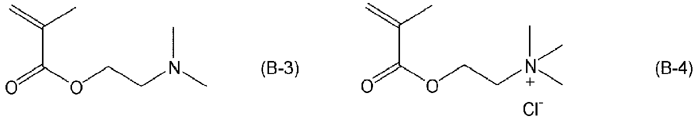

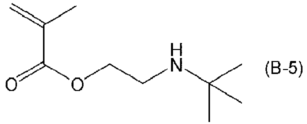

The monomer mixture according to the present invention contains a hydrophobic monomer represented by the formula (C): 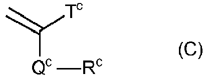 [wherein
T^{c} represents a hydrogen atom or a linear or branched alkyl group having 1 to 5 carbon atoms;
Q^{c} represents a single bond, an ether bond or an ester bond;
R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms (where the aryl portion may be substituted by a linear or branched alkyl group having 1 to 5 carbon atoms which may be substituted by a halogen atom(s),)].
It may contain two or more kinds of the hydrophobic monomers represented by the formula (C).

In one embodiment of the hydrophobic monomer represented by the formula (C), as T^{c}, a hydrogen atom, a methyl group or an ethyl group is preferred, and a hydrogen atom or a methyl group is more preferred. As Q^{c}, a single bond or an ester bond is preferred, and an ester bond is more preferred. As R^{c}, a linear or branched alkyl group having 1 to 18 carbon atoms or a cyclic hydrocarbon group having 3 to 10 carbon atoms is preferred, and a linear or branched alkyl group having 1 to 6 carbon atoms or a cycloalkyl group having 3 to 10 carbon atoms is more preferred.

Specific examples of the monomer of the above-mentioned formula (C) may be mentioned a linear or branched alkyl esters of (meth)acrylic acids such as butyl (meth)acrylate, 2-ethylhexyl (meth)acrylate, lauryl (meth)acrylate, stearyl (meth)acrylate, etc.; cyclic alkyl esters of (meth)acrylic acids such as cyclohexyl (meth)acrylate, isobornyl (meth)acrylate, etc.; aralkyl esters of (meth)acrylic acids such as benzyl (meth)acrylate, phenethyl (meth)acrylate, etc.; styrene-based monomers such as styrene, methylstyrene, chloromethylstyrene, etc.; vinyl ether-based monomers such as methyl vinyl ether, butyl vinyl ether, etc.; and vinyl ester-based monomers such as vinyl acetate, vinyl propionate, etc. Among these, butyl (meth)acrylate or cyclohexyl (meth)acrylate is preferably used.

The structural formulae of butyl methacrylate (=butyl methacrylate) and cyclohexyl methacrylate (=methacrylic acid cyclohexyl) are represented by the following formula (C-1) and formula (C-2), respectively.

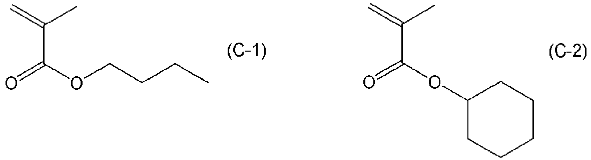

The monomer mixture according to the present invention contains a bifunctional monomer represented by the formula (D): 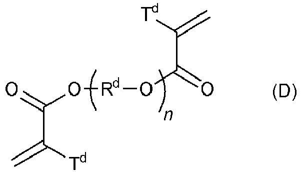 (wherein
T^{d} represents a hydrogen atom or a linear or branched alkyl group having 1 to 6 carbon atoms;
R^{d} represents a linear or branched alkylene group having 1 to 10 carbon atoms which may be substituted by a halogen atom(s); and
n represents an integer of 1 to 10).
It may contain two or more kinds of the bifunctional monomers represented by the formula (D).

In one embodiment of the bifunctional monomer represented by the formula (D), as T^{d}, a hydrogen atom, a methyl group or an ethyl group is preferred, and a hydrogen atom or a methyl group is more preferred. As R^{d}, a methylene group, an ethylene group or a propylene group each of which may be substituted by a chlorine atom is preferred, and an ethylene group or a propylene group is more preferred. n is preferably 1 to 8.

Specific examples of the bifunctional monomer of the above-mentioned formula (D) may be mentioned poly(ethylene glycol) di(meth)acrylate, poly(trimethylene glycol) di(meth)acrylate, polypropylene glycol) di(meth)acrylate, etc.

For example, the structural formulae of ethylene glycol dimethacrylate, poly(ethylene glycol) dimethacrylate and polypropylene glycol) dimethacrylate are represented by the following formula (D-1) to formula (D-3), respectively. 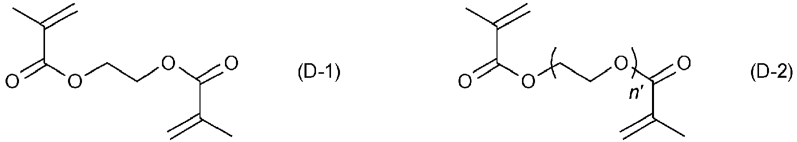 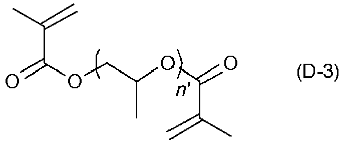 n'=1-6

In the monomer mixture according to the present invention, a ratio of the total of the above-mentioned anionic monomer represented by the formula (A) and the above-mentioned cationic monomer represented by the formula (B) based on the total monomers is 40 mol% or more, preferably 40 mol% or more and 70 mol% or less, more preferably 40 mol% or more and 60 mol% or less, and particularly preferably 40 mol% or more and 55 mol% or less. In addition, a ratio (molar ratio) of the above-mentioned anionic monomer represented by the formula (A) and the above-mentioned cationic monomer represented by the formula (B) is not particularly limited, and preferably in the range of 1:2 to 2:1, more preferably in the range of 1:1.5 to 1.5:1, and particularly preferably in the range of 1:1.2 to 1.2:1.

In the monomer mixture according to the present invention, a ratio of the bifunctional monomer represented by the above-mentioned formula (D) based on the total monomers is preferably less than 30 mol%, preferably 5 mol% or more and less than 30 mol%, more preferably 10 mol% or more and less than 30 mol%, and particularly preferably 15 mol% or more and less than 30 mol%. In particular, in the coating film of the present invention, when post-crosslinking by a crosslinking agent is expected, it is possible to reduce the ratio of the bifunctional monomer in the monomer mixture.

In the monomer mixture according to the present invention, a ratio of the hydrophobic monomer represented by the above-mentioned formula (C) based on the total monomers may be all the remainder after deducting the ratio of the monomers of the above-mentioned formula (A), (B) and (D) from the total monomers, or may be the remainder after deducting the total ratio of the monomers of the above-mentioned formula (A), (B) and (D) and the following optional monomer component, and for example, it is 1 mol% or more and 55 mol% or less, preferably to be 3 mol% or more and 50 mol% or less, and more preferably to be 5 mol% or more to 50 mol% or less.

The monomer mixture according to the present invention may further contain an ethylenically unsaturated monomer, or a repeating unit derived from a polysaccharide or a derivative thereof as an optional monomer component. Examples of the ethylenically unsaturated monomer may be mentioned one or two or more kinds of ethylenically unsaturated monomers selected from the group consisting of (meth)acrylic acid ester; vinyl acetate; vinyl pyrrolidone; ethylene; vinyl alcohol; and a hydrophilic functional derivative thereof. Examples of the polysaccharide or a derivative thereof may be mentioned cellulose-based polymers such as hydroxyalkyl cellulose (for example, hydroxyethyl cellulose or hydroxypropyl cellulose), etc., starch, dextran and curdlan.

As the hydrophilic functional derivative refers to an ethylenically unsaturated monomer having a hydrophilic functional group or structure. Examples of the hydrophilic functional group or structure may be mentioned may be mentioned a betaine structure; an amide structure; an alkylene glycol residue; an amino group; and a sulfinyl group, etc.

The betaine structure means a monovalent or divalent group of a compound having an amphoteric center with a quaternary ammonium type cationic structure and an acidic anionic structure, and, for example, there may be mentioned a phosphoryl-choline group: 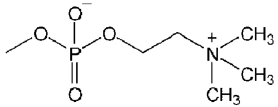 Examples of the ethylenically unsaturated monomer having such a structure may be mentioned 2-methacryloyloxyethylphosphorylcholine (MPC), etc.

The amide structure means a group represented by the following formulae: 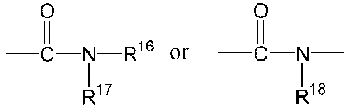 [here, R¹⁶, R¹⁷ and R¹⁸ are each independently a hydrogen atom or an organic group (for example, a linear or branched alkyl group having 1 to 5 carbon atoms which may be substituted, etc., and specifically a methyl group, an isopropyl group, a hydroxymethyl group or a hydroxyethyl group, etc.)].
Examples of the ethylenically unsaturated monomer having such a structure may be mentioned, (meth)acrylamide, N-isopropylacrylamide, N-(hydroxymethyl) (meth)acrylamide, etc. Further, the monomers having such a structure are disclosed in, for example, JP 2010-169604A, etc.

The alkylene glycol residue means an alkyleneoxy group (-Alk-O-) which remains after condensation reaction of hydroxy groups at one terminal or both terminals of alkylene glycol (HO-Alk-OH; here, Alk is an alkylene group having 1 to 10 carbon atoms) are reacted with other compounds, and also includes a poly(alkyleneoxy) group in which the alkyleneoxy unit is repeated. Examples of the ethylenically unsaturated monomer having such a structure may be mentioned 2-hydroxyethyl (meth)acrylate, methoxypolyethylene glycol (meth)acrylate, etc. Further, the monomers having such a structure are disclosed in, for example, JP 2008-533489 A, etc.

The amino group means a group represented by the formula: -NH₂, -NHR¹⁹ or -NR²⁰R²¹ [here, R¹⁹, R²⁰ and R²¹ are each independently an organic group (for example, a linear or branched alkyl group having 1 to 5 carbon atoms, etc.)]. In the amino group of the present invention, a quaternarized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 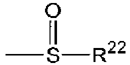 [here, R²² is an organic group (for example, an organic group having 1 to 10 carbon atoms, and preferably an alkyl group having 1 to 10 carbon atoms and having one or more hydroxy groups, etc.)].
As an introducing method of the sulfinyl group, there may be mentioned a method disclosed in JP 2014-48278A, etc.

A weight average molecular weight of the polymer contained in the composition for forming a coating film of the present invention may be from several thousands to several million, preferably from 5,000 to 5,000,000, and further preferably 10,000 to 2,000,000. In addition, the polymer may be either of a random copolymer, a block copolymer or a graft copolymer, and a random copolymer is preferred.

The polymer contained in the composition for forming a coating film of the present invention can be obtained by polymerizing the monomer mixture which contains monomers represented by the above-mentioned formulae (A) to (D) (and occasionally an optional monomer component(s)). Polymerization can be carried out by a method known *per se* (for example, the method described in JP 2014-162865A or WO 2020/040247). For example, it can be synthesized by methods such as radical polymerization, anionic polymerization, cationic polymerization, etc., which are general synthetic methods for acrylic polymers or methacrylic polymers, etc. As its forms, various methods such as solution polymerization, suspension polymerization, emulsion polymerization, bulk polymerization, etc., are possible.

Polymerization can be carried out, for example, by a production method including a step of reacting (polymerizing) the monomers represented by the above-mentioned formulae (A) to (D) in a solvent.

The reaction conditions are such that a reaction vessel to which various raw materials (monomer, solvent, initiator, etc.) are added is heated to 50°C to 200°C in an oil bath, etc., and stirred for 1 hour to 48 hours, and more preferably at 80°C to 150°C, 5hours to 30 hours, whereby the polymerization reaction proceeds and the copolymer according to the present invention is obtained. The reaction atmosphere is preferably a nitrogen atmosphere.

As the solvent in the polymerization reaction may be water, a phosphate buffer, an alcohol such as ethanol, etc., or a mixed solvent in which these are combined, and it is desirable to contain water or ethanol. Further, it is preferred to contain 10 % by mass or more and 100 % by mass or less of water or ethanol. Furthermore, it is preferred to contain 50 % by mass or more and 100 % by mass or less of water or ethanol. Moreover, it is preferred to contain 80 % by mass or more and 100 % by mass or less of water or ethanol. Still further, it is preferred to contain 90 % by mass or more and 100 % by mass or less of water or ethanol. Preferably, the sum of water and ethanol is 100% by mass.

As a reaction procedure, all raw materials may be put into a reaction solvent at room temperature and then heated to the above-mentioned temperature to polymerize, or all or part of the mixture of raw materials may be added dropwise little by little into a preheated solvent. For example, the anionic monomer represented by the formula (A) is a monomer that easily associates, it may be added dropwise to the reaction solvent little by little so that it can be quickly dispersed when dropped into the reaction system. In this case, the reaction solvent may be heated (for example, 40°C to 100°C) to increase the solubility of the monomer and polymer.

In order to proceed the polymerization reaction efficiently, it is desirable to use a polymerization initiator, particularly a radical polymerization initiator. Examples of the radical polymerization initiator may be mentioned azo polymerization initiators such as dimethyl 1,1'-azobis(1-cyclohexanecarboxylate) (VE-073, available from FUJIFILM Wako Pure Chemical Corporation), 2,2'-azobis(2,4-dimethylvaleronitrile) (V-65, available from FUJIFILM Wako Pure Chemical Corporation), 2,2'-azobis(isobutyronitrile) (AIBN, available from FUJIFILM Wako Pure Chemical Corporation), 2,2'-azobis[N-(2-carboxyethyl)-2-methylpropionamidine] n hydrate (VA-057, available from FUJIFILM Wako Pure Chemical Corporation), 2,2' -(N-butyl-2-methyl-propionamide) (VAm-110, available from FUJIFILM Wako Pure Chemical Corporation), etc.

An amount of the polymerization initiator is 0.05% by mass to 10% by mass based on the total weight of the monomers to be used for polymerization.

After completion of the reaction, the obtained polymer may be isolated and purified by a known method, for example, by adding a poor solvent to the reaction solution, but the reaction solution may be used for preparation of the composition for forming a coating film of the present invention as the copolymer-containing solution as such.

### (Polycarbodiimide)

The polycarbodiimide of the present invention contains the structure represented by the following formula (E):

-N=C=N- (E)

It can be considered that the reaction of the polymer according to the present invention and polycarbodiimide proceeds according to the following formula. As shown in this reaction formula, carbodiimide accelerates dehydration condensation between phosphoric acid groups, whereby a pyrophosphate structure is formed between the polymers, and crosslinking reaction (curing) of the polymer thought to promote.

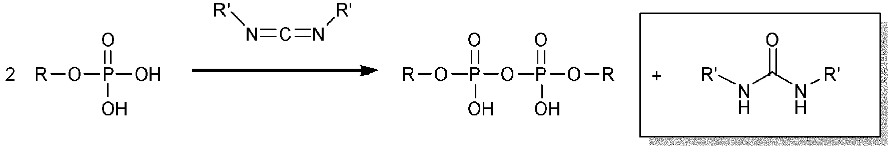

The polycarbodiimide of the present invention may be a polycarbodiimide compound derived from an aliphatic diisocyanate compound having at least one primary isocyanate group, and
the above-mentioned polycarbodiimide compound may have a structure in which it is sealed with an organic compound(s) having a functional group which reacts with an isocyanate group at all ends.

For example, the above-mentioned aliphatic diisocyanate compound having at least one primary isocyanate group may be at least one kind selected from the group consisting of a linear aliphatic isocyanate compound such as ethylene diisocyanate, tetramethylene diisocyanate, hexamethylene diisocyanate, 2,2,4-trimethylhexamethylene diisocyanate, etc., a cyclic aliphatic diisocyanate compound such as isophorone diisocyanate, 1,3-bis(isocyanatomethyl)cyclohexane, norbornane diisocyanate, etc., and an aliphatic diisocyanate compound having an aromatic ring such as xylylene diisocyanate, etc.

The polycarbodiimide of the present invention may be, for example, an isocyanate-terminated polycarbodiimide having at least two carbodiimide groups represented by the above-mentioned formula (E) which is obtained by condensation reaction accompanied by removal of carbon dioxide from the above-mentioned aliphatic diisocyanate compound having at least one primary isocyanate group, or may be a polycarbodiimide having a structure in which the terminal is sealed with an organic compound having a functional group that reacts with the isocyanate group.

The above-mentioned functional group possessed by the above-mentioned organic compound may be at least one kind selected from a hydroxy group, an amino group, an isocyanate group, an epoxy group and a carboxy group.

Examples of the above-mentioned organic compound may be mentioned an organic compound having a hydroxy group(s) such as polyethylene glycol monomethyl ether, polyethylene glycol monoethyl ether, polypropylene glycol monomethyl ether, polypropylene glycol monoethyl ether, triethylene glycol monomethyl ether, triethylene glycol monoethyl ether, tetraethylene glycol monomethyl ether, tetraethylene glycol monoethyl ether, diethylene glycol monomethyl ether, diethylene glycol monoethyl ether, ethyl alcohol, propyl alcohol, butyl alcohol, pentyl alcohol, hexyl alcohol, octyl alcohol, dodecyl alcohol, etc.; an organic compound having an amino group(s) such as methylamine, ethylamine, propylamine, butylamine, pentylamine, hexylamine, octylamine, dodecylamine, diethylamine, dipropylamine, dibutylamine, cyclohexylamine, adamantaneamine, allylamine, polyoxyethylene laurylamine, polyoxymethylene stearylamine, aniline, diphenylamine, 3-aminopropyltrimethoxysilane, 3-aminopropyltriethoxysilane, N-phenyl-3-aminopropyltrimethoxysilane, 2,2-difluoroamine, fluorobenzylamine, trifluoroethylamine and [[4-(trifluoromethyl)cyclohexyl]methyl]amine or a derivative(s) thereof, etc.; an organic compound having an isocyanate group(s) such as butyl isocyanate, pentyl isocyanate, hexyl isocyanate, octyl isocyanate, dodecyl isocyanate, cyclohexyl isocyanate, 1-adamantyl isocyanate, 3-isocyanatepropyltriethoxysilane, acrylic acid 2-isocyanate ethyl, benzyl isocyanate, 2-phenylethylisocyanate or a derivative(s) thereof, etc.; an organic compound having an epoxy group such as 1,2-epoxyheptane, 1,2-epoxyhexane, 1,2-epoxydecane, 1,2-epoxy-5-hexene, ethyl glycidyl ether, 2-ethylhexyl glycidyl ether, glycidyl lauryl ether, allyl glycidyl ether, diethoxy(3-glycidyloxypropyl)methylsilane, 3-[2-(perfluorohexyl)ethoxy]-1,2-epoxypropane or a derivative(s) thereof, etc.; an organic compound having a carboxy group such as acetic acid, ethanoic acid, propionic acid, butanoic acid, pentanoic acid, hexanoic acid, heptanoic acid, octanoic acid, nonanoic acid, decanoic acid, cyclohexanecarboxylic acid, adamantaneacetic acid, phenylacetic acid, benzoic acid, undecenoic acid or a derivative(s) thereof, etc.

The above-mentioned organic compound may further have a hydrophilic group(s) other than the above-mentioned functional group(s).

It is preferred that the above-mentioned polycarbodiimide contains a hydrophilic group(s).

It is preferred that the above-mentioned hydrophilic group is represented by the following formula (F):

R¹-(O-CHR²-CH₂)ₒ- (F)

(wherein
R¹ represents a linear or branched alkyl group having 1 to 5 carbon atoms,
R² represents a hydrogen atom or a methyl group, and when a plural number of R² are present, said R²s may be the same or different from each other, and o represents an integer of 1 to 30).

As the organic compound having a functional group which reacts with an isocyanate group and a hydrophilic group, in particular, a hydrophilic group represented by the above-mentioned formula (F), there may be mentioned, for example, polyethylene glycol monomethyl ether (MPEG), tetraethylene glycol monomethyl ether (MTEG), etc., these may be used alone or may be used in combination of two or more kinds. By sealing the terminal of the isocyanate-terminated polycarbodiimide with such an organic compound, it is possible to obtain the polycarbodiimide into which the hydrophilic group represented by the above-mentioned formula (F) is introduced.

As the above-mentioned polycarbodiimide, a polycarbodiimide compound described in WO 2018/194102 can be used. With regard to the other details of the polycarbodiimide compound of the present invention, these conform to the contents described in WO 2018/194102.

As the above-mentioned polycarbodiimide, a commercially available product may be used. As the trade name of the commercially available product, there may be mentioned, for example, "Carbodilite V-02," "Carbodilite V-02-L2," "Carbodilite SV-02," "Carbodilite V-04," "Carbodilite V-10," "Carbodilite E-02" and "Carbodilite E-05" (each available from Nisshinbo Chemical Inc., trade names).

### (Composition for forming coating film)

The composition for forming a coating film of the present invention can be obtained by mixing the above-mentioned polymer and polycarbodiimide. A ratio of the polycarbodiimide to the polymer is, for example, based on 100 parts by weight of the polymer, in the range of 1 to 30 parts by weight, preferably in the range of 1 to 20 parts by weight, more preferably in the range of 3 to 20 parts by weight, and particularly preferably in the range of 5 to 20 parts by weight. Incidentally, when the polymer according to the present invention contains the bifunctional monomer, the ratio of the polycarbodiimide based on the polymer in the composition for forming a coating film may be also reduced.

The composition for forming a coating film of the present invention contains a solvent in addition to the above-mentioned polymer and polycarbodiimide. The solvent may be derived from the reaction solution of the copolymer or may be added separately.

As the solvent contained in the composition for forming a coating film of the present invention, there may be mentioned water, phosphate buffered saline phosphate buffered physiological saline (PBS) and an alcohol. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, and these may be used alone or a mixed solvent in combination with these, but from the viewpoint of dissolving the copolymer, it is preferred to be selected from water, PBS, ethanol and propanol.

The concentration of solids in the composition for forming a coating film according to the present invention is desirably 0.01 to 50% by mass in order to form a coating film uniformly.

Further, to the composition for forming a coating film of the present invention, other substance(s) may be added in a range that does not impair the properties of the obtainable coating film, if necessary, in addition to the above-mentioned polymer and polycarbodiimide, and the solvent. As the other substance(s), there may be mentioned preservatives, surfactants, primers to improve adhesion to base materials, fungicides and sugars, etc.

In order to adjust the ion balance of the copolymer in the composition for forming a coating film according to the present invention, at the time of obtaining the coating film of the present invention, a step of adjusting pH in the composition for forming a coating film may be further contained in advance. The pH adjustment may be carried out by, for example, adding a pH adjusting agent to the composition containing the above-mentioned copolymer and solvent, and making the pH of the said composition 3.0 to 13.5, preferably 3.5 to 8.5, further preferably 3.5 to 5.5, or preferably 8.5 to 13.5, and further preferably 10.0 to 13.5. A kind and an amount of the pH adjusting agent that can be used are appropriately selected according to the concentration of the above-mentioned copolymer, and the existence ratio of the anions and cations, etc.

Examples of the pH adjusting agent may be mentioned an organic amine such as ammonia, triethylamine, diethanolamine, pyridine, N-methyl-D-glucamine, tris(hydroxymethyl)aminomethane, etc.; an alkali metal hydroxide such as potassium hydroxide, sodium hydroxide, etc.; an alkali metal halide such as potassium chloride, sodium chloride, etc.; an inorganic acid or an alkali metal salt thereof such as sulfuric acid, phosphoric acid, hydrochloric acid, carbonic acid, etc.; a quaternary ammonium cation such as choline, etc., or a mixture thereof (for example, a buffer such as phosphate buffered physiological saline, etc.). Among these, ammonia, triethylamine, diethanolamine, sodium hydroxide, choline, N-methyl-D-glucamine and tris(hydroxymethyl)aminomethane are preferred, and particularly ammonia, triethylamine, diethanolamine, sodium hydroxide and choline are preferred.

A second embodiment of the composition for forming a coating film of the present invention includes a monomer mixture containing an anionic monomer represented by the formula (A), a cationic monomer represented by the formula (B), and a hydrophobic monomer represented by the formula (C), a polymer of the monomer mixture wherein a ratio of the sum of the anionic monomer represented by the formula (A) and the cationic monomer represented by the formula (B) based on the total monomers contained in the above-mentioned monomer mixture is 40 mol% or more, and a polycarbodiimide containing a structure represented by the formula (E).

The descriptions such as explanation and preferred embodiment with regard to the monomers represented by the formulae (A) to (C) and its polymer, a polycarbodiimide containing a structure represented by the formula (E), and the composition for forming a coating film, etc., in the first embodiment are applied to the second embodiment except for the bifunctional monomer represented by the formula (D).

A third embodiment of the composition for forming a coating film of the present invention is a monomer mixture containing, among the above-mentioned polymer, an anionic monomer represented by the formula (A), a cationic monomer represented by the formula (B), a hydrophobic monomer represented by the formula (C), and a bifunctional monomer represented by the formula (D), which contains a polymer of the monomer mixture wherein a ratio of the total of the anionic monomer represented by the formula (A) and the cationic monomer represented by the formula (B) based on the total monomers contained in the above-mentioned monomer mixture is 40 mol% or more, and does not contain a polycarbodiimide.

The descriptions such as explanation and preferred embodiment with regard to the monomers represented by the formulae formula (A) to (D) and its polymer, and the composition for forming a coating film, etc., in the first embodiment are applied to the third embodiment except for the polycarbodiimide.

### <Coating film/cured product>

The first or the second embodiment of the coating film of the present invention is a cured product of an applied film of the composition for forming a coating film in the above-mentioned first or second embodiment, respectively. The cured product can be formed by applying the composition for forming a coating film according to the present invention onto at least part of the surface of a substrate, and drying the same. The applying method is not particularly limited, and usual applying methods such as spin coating, dip coating, spray coating, solvent casting, etc., are used.

As a specific applying method, for example, there may be used methods such as dipping a substrate in the above-mentioned composition for forming a coating film as mentioned later, adding the composition for forming a coating film to a container and allowing to stand for a predetermined period of time, or applying the composition for forming a coating film onto a surface of a container or a substrate, etc., and in the case of a container, as an embodiment, a cell culture container, it is carried out by the method of adding the composition for forming a coating film to a container and allowing to stand for a predetermined period of time. Addition can be carried out, for example, by adding the composition for forming a coating film in an amount of 0.5 to 1 times the total volume of the container using a syringe, etc. Standing is practiced depending on the material of the substrate or components of the composition for forming a coating film appropriately selecting the time and temperature and, for example, it is practiced from 1 minute to 24 hours, preferably from 5 minutes to 3 hours at 10 to 80°C. According to this procedure, an applied film can be formed onto at least part of the surface of the container, and preferably over the entire surface.

Then, the applied film is subjected to a drying step to form a cured product. The drying step is carried out under atmosphere or under vacuum at a temperature within the range of -200°C or higher and lower than 200°C. The cured product can be formed by, for example, drying at room temperature (10°C to 35°C, for example, 25°C), but in order to form a cured product coating film more rapidly, it may be dried, for example, at 40°C to 100°C. In addition, a drying step at extremely low to low temperatures (around -200°C to -30°C) by a freeze-drying method may be used. Freeze-drying is called as vacuum freeze-drying, and is a method in which a substance to be dried is usually cooled with a refrigerant and the solvent is removed by sublimation in a vacuum state. Common refrigerants used in freeze-drying may be mentioned a mixed medium of dry ice and methanol (-78°C), liquid nitrogen (-196°C), etc. According to this drying step, the anionic groups of the polymer causes a crosslinking reaction through the polycarbodiimide compound, for example, as shown in the above-mentioned reaction formula, to become a cured product whereby a coating film can be formed.

The coating film of the present application may be a material obtained by further washing after forming a cured product by the above-mentioned steps.

The above-mentioned washing may be carried out by a known method, and it is preferable to use running water washing, ultrasonic washing, etc. As the washing solvent, there may be mentioned water, an aqueous solution containing an electrolyte(s), and an alcohol. Here, the aqueous solution containing an electrolyte(s) is preferably PBS, physiological saline (containing sodium chloride alone), Dulbecco's phosphate-buffered physiological saline, tris buffered physiological saline, HEPES buffered physiological saline and Veronal-buffered physiological saline, and PBS is particularly preferred. The alcohol is preferably an alcohol having 2 to 6 carbon atoms, and ethanol is particularly preferred. As the washing solvent, a water-containing alcohol solvent, which is a mixture of water or an aqueous solution containing electrolyte(s) and an alcohol, is preferable, and a water-containing ethanol solvent, which is a mixture of water and ethanol, is more preferable. The washing solvent is usually used at room temperature (for example, 10 to 35°C) and it may be a material heated in the range of, for example, 40°C to 95°C. After being fixed, the coating film is not dissolved out even when it is washed with water, PBS and an alcohol, etc., and remains firmly fixed to the substrate, and it has the effect that the film thickness changes little before and after washing, i.e., the coating film is less dissolved out into the solvent.

A film thickness of the coating film of the present invention is in the range of 1 to 1,000 nm, and preferably in the range of 5 to 500 nm, 10 to 300 nm, 10 to 200 nm, 10 to 100 nm and 10 to 50 nm.

A third embodiment of the coating film of the present invention is an applied film of the composition for forming a coating film mentioned in the above-mentioned third embodiment. The coating film can be formed by applying the composition for forming a coating film according to the present invention onto at least part of a surface of a substrate and drying the same.

The descriptions such as explanation and preferred embodiment with regard to the process for producing the coating film in the first embodiment, in particular, the applying method of the composition for forming a coating film and the drying and washing steps, etc., in the first embodiment are applied to the third embodiment except for those relating to curing.

### (Substrate)

The substrate referred to the present invention may be, in addition to a flat plate substrate having a flat surface, a container, an instrument, etc., having an arbitrary structure. Examples of such a substrate may be instruments for collecting or delivering the above-mentioned biological substances (for example, blood glucose meter, injection needle, catheter, etc.), containers for storing the above-mentioned biological substances (for example, bags, bottles, vials, etc., and specifically blood bags, storage containers of antibody drug, etc.), instruments for separating, isolating or analyzing the above-mentioned biological substances (for example, microscope peripheral equipment such as carriers, cover glasses, etc., microfluidic devices including flow cytometers such as cell sorters, etc., cuvettes, substrates for cell culture, cell spheroid arrays, cell separation columns, microchannel chips, microwell array chips, assay chips, biochips, magnetic beads, measurements for fully automated analyzers cells, etc.), instruments for bioprocessing (for example, reaction vessels, transfer tubes, transfer pipes, instruments for purification, cell culture plates, etc.), prosthetic materials (for example, implants, bone fixation materials, sutures, anti-adhesion membranes, artificial blood vessels etc.) as well as drug delivery vehicles such as vesicles, microparticles, nanoparticles, etc., materials for diagnostic instruments such as gastroscopes, etc., and materials for medical applications such as microfibers, nanofibers, magnetic particles, etc.

By applying the above-mentioned composition for forming a coating film onto the surface of a substrate and drying it, a substrate capable of suppressing the adhesion of biological substances can be produced. Here, the "surface" refers to the surface that comes into contact with the biological substances.

The substrate for cell culture is a substrate for cell culture having the above-mentioned coating film onto at least part of the substrate surface on the substrate. It is preferable that the above-mentioned coating film is formed over the entire surface on which cell culture is carried out.

In particular, as the substrate for cell culture, there may be mentioned, for example, dishes (petri dishes) generally used for culture of cells such as petri dishes, dishes for tissue culture, multi-dishes, etc., flasks such as flasks for cell culture, spinner flasks, etc., bags such as plastic bags, Teflon (Registered Trademark) bags, culture bags, etc., plates such as microplates, microwell plates, multiplates, multiwell plates, etc., chamber slides, tubes, trays, bottles such as roller bottles, etc., culture containers having a stirring blade inside thereof for stirring a cell suspension, large-sized culture vessels, bioreactors, etc. It is preferably mentioned dishes, plates and trays.

In addition, the material of the substrate may be mentioned, for example, glass, metal, metal-containing compound or semimetal-containing compound, activated carbon or resin. The metals may be mentioned typical metals: (alkali metals: Li, Na, K, Rb, Cs; alkaline earth metals: Ca, Sr, Ba, Ra), magnesium group elements: Be, Mg, Zn, Cd, Hg; aluminum group elements: Al, Ga, In; rare earth elements: Y, La, Ce, Pr, Nd, Sm, Eu; tin group elements: Ti, Zr, Sn, Hf, Pb, Th; iron group elements: Fe, Co, Ni; earth-acid element: V, Nb, Ta, chromium group elements: Cr, Mo, W, U; manganese group elements: Mn, Re; noble metal: Cu, Ag, Au; platinum group elements: Ru, Rh, Pd, Os, Ir, Pt, etc. The metal-containing compound or the semimetal-containing compound may be mentioned, for example, ceramics, which are sintered bodies whose basic components are metal oxides and are sintered by heat treatment at high temperatures, semiconductors such as silicon, inorganic solid materials such as molded bodies of inorganic compounds, etc., such as metal oxide or semimetal oxide (silicon oxide, alumina, etc.), metal carbide or semimetal carbide, metal nitride or semimetal nitride (silicon nitride, etc.), metal boride or semimetal boride, etc., aluminum, nickel titanium, stainless steel (SUS304, SUS316, SUS316L, etc.).

As the resins, it may be either of a natural resin or a derivative thereof, or a synthetic resin, as the natural resin or a derivative thereof, there may be mentioned cellulose, cellulose triacetate (CTA), nitrocellulose (NC), cellulose to which dextran sulfate is immobilized, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol(PVA), polyurethane (PU), ethylene vinyl alcohol (EVAL), polyethylene(PE), poly ester, polypropylene (PP), polyvinylidene fluoride (PVDF), polyether sulfone (PES), polycarbonate (PC), polyvinyl chloride (PVC), polytetrafluoroethylene (PTFE), ultra-high molecular weight polyethylene (UHPE), cycloolefin polymer (COP), cycloolefin copolymer (COC), polydimethylsiloxane (PDMS), acrylonitrile-butadiene-styrene resin (ABS) or Teflon (Registered Trademark). In the production of the substrates for cell culture of the present invention, it is not necessary to require the treatment at high temperature when the composition for forming a coating film is subjected to coating so as to present onto at least part of the surface of the substrate, so that a resin having low heat resistance, etc., can be also applied.

The material of the substrate may be one kind or a combination of two or more kinds. Among these materials, it is preferably glass, silicon, silicon oxide, polystyrene (PS), polypropylene (PP), poly ether sulfone (PES), polyethylene terephthalate (PET), polycarbonate (PC), polyvinyl chloride (PVC), Teflon (Registered Trademark), cycloolefin polymer (COP), cycloolefin copolymer (COC), polydimethylsiloxane (PDMS) or stainless steel (SUS304, SUS316, SUS316L, etc.) alone, or a combination selected therefrom, and particularly preferably glass, polystyrene (PS), polypropylene (PP), stainless steel (SUS304, SUS316, SUS316L, etc.), cycloolefin polymer (COP), cycloolefin copolymer (COC) and polydimethylsiloxane (PDMS).

### (Biological substances)

The coating film of the present invention is preferably to be a coating film having an ability to suppress adhesion of biological substances. Similarly, the composition for forming a coating film of the present invention is preferably to be a composition for forming a coating film having an ability to suppress adhesion of biological substances.

In the present invention, as the biological substances, there may be mentioned proteins, sugars, viruses, nucleic acids and cells, or combinations thereof, or biological tissues and body fluids containing these.

As the above-mentioned proteins, there may be mentioned fibrinogen, bovine serum albumin (BSA), human albumin, various kinds of globulins, β-lipoproteins, various kinds of antibodies (IgG, IgA and IgM), peroxidase, various kinds of complements, various kinds of lectins, fibronectin, lysozyme, von Willebrand factor (vWF), serum γ-globulin, pepsin, ovalbumin, insulin, histone, ribonuclease, collagen and cytochrome c,
as the above-mentioned sugars, glucose, galactose, mannose, fructose, heparin and hyaluronic acid,
as the above-mentioned nucleic acids, deoxyribonucleic acid (DNA) and ribonucleic acid (RNA),
as the above-mentioned cells, fibroblasts, bone marrow cells, B lymphocytes, T lymphocytes, neutrophils, red blood cells, platelets, macrophages, monocytes, osteocytes, pericytes, dendrites, keratinocytes, adipocytes, mesenchymal cells, epithelial cells, epidermal cells, endothelial cells, vascular endothelial cells, hepatocytes, chondrocytes, cumulus cells, nervous system cells, glial cells, neurons, oligodendrocytes, micro glia, astrocytes, cardiac cells, esophageal cells, muscle cells (for example, smooth muscle cells or skeletal muscle cells), pancreatic beta cells, melanocytes, hematopoietic progenitor cells, mononuclear cells, embryonic stem cells (ES cells), embryonic tumor cells, embryonic germ stem cells, induced pluripotent stem cells (iPS cells), neural stem cells, hematopoietic stem cells, mesenchymal stem cells, liver stem cells, pancreatic stem cells, muscle stem cells, germ stem cells, intestinal stem cells, cancer stem cells, hair Follicle stem cells and various kinds of cell lines (for example, HCT116, Huh7, HEK293 (human embryonic kidney cells), HeLa (human cervical cancer cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns0/1, Jurkat, NIH3T3, PC12, S2, Sf9, Sf21, High Five, Vero), etc.

In addition, the biological substances in the present invention may be a substance that can act upon being administered to a living body. Examples of such a substance may be mentioned peptides (cyclic peptides), low molecular weight medicines such as low molecular weight compounds, etc., biopharmaceuticals such as enzymes, blood coagulation fibrinolytic factors, serum proteins, hormones, vaccines, interferons, erythropoietins, cytokines, toxins, antibodies, antibody drug conjugates, fusion proteins, etc.

### <Storage container for antibody drug>

The coating film of the present invention is also preferably a coating film having an ability to suppress aggregation of antibodies. Similarly, the composition for forming a coating film of the present invention is preferably a composition for forming a coating film having an ability to suppress adhesion such as an ability to suppress aggregation of antibody.

Accordingly, the present invention relates to, in particular, a storage container of an antibody drug having any of the coating film mentioned in the above-mentioned first to third embodiments at least part of the surface thereof. The form and material of the storage container are as mentioned in the section of the above-mentioned (Substrate).

An antibody drug is a drug using an antibody, and the antibody is a protein composed of immunoglobulin.

It is preferred that the above-mentioned antibody drug contains at least one of an antibody and an antigen-binding fragment thereof.

It is preferred that the above-mentioned antibody drug contains at least one selected from the group consisting of chimeric antibodies, human antibodies, humanized antibodies, and domain antibodies thereof.

Specific examples the above-mentioned antibody drug may be mentioned ofatumumab (Product name "Azera (Registered Trademark)"), cetuximab (Product name "Erbitux (Registered Trademark)"), tocilizumab (Product name "Actemra (Registered Trademark)"), bevacizumab (Product name "Avastin (Registered Trademark)"), canakinumab (Product name "Ilaris (Registered Trademark)"), golimumab (Product name "Simponi (Registered Trademark)"), Ustekinumab (Product name "Stelara (Registered Trademark)"), Eculizumab (Product name "Soliris (Registered Trademark)"), Omalizumab (Product name "Xolair (Registered Trademark)"), Trastuzumab (Product name "Herceptin (Registered Trademark)"), Pertuzumab (Product name "Perjeta (Registered Trademark)"), adalimumab (Product name "Humira (Registered Trademark)"), denosumab (Product name "Pralia (Registered Trademark)," "Ranmark (Registered Trademark)"), mogamulizumab (Product name "Poteligeo (Registered Trademark)"), rituximab (Product name "Rituxan (Registered Trademark)"), ranibizumab (Product name "Lucentis (Registered Trademark)"), infliximab (Product name "Remicade (Registered Trademark)"), aflibercept (Product name "Eylea (Registered Trademark)"), abatacept (Product name "Orencia (Registered Trademark)"), etanercept (Product name "Enbrel (Registered Trademark)"), gemtuzumab ozogamicin (Product name "Mylotarg (Registered Trademark)"), panitumumab (Product name "Vectibix (Registered Trademark)"), basiliximab (Product name "Symlect (Registered Trademark)"), certolizumab pegol (Product name "Cimzia (Registered Trademark)"), palivizumab (Product name "Synagis (Registered Trademark)"), casilibimab/imdevimab (Product name "Lonaprive (Registered Trademark)") and sotrovimab (Product name "Xebdy").

Among these, it is preferred to contain an antibody obtained from a clone derived from a single antibody-producing cell, or a monoclonal antibody that is an antibody molecule, it is preferred to contain a monoclonal antibody comprising an antihuman CD20 human antibody, and it is preferred to contain rituximab or abatacept.

Incidentally, the antibody drug may contain one kind of an antibody or may contain two or more kinds of antibodies. That is, the antibody drug of the present invention may contain both an antibody and an antigen-binding fragment, may contain two or more kinds of antibodies, or may contain two or more kinds of antigen-binding fragments.

Having an ability to suppress adhesion of protein means that the relative average absorbance (%) ((average absorbance of Example)/(average absorbance without the uncoated film)) compared with the case of no coating film by the IgG antibody HRP measurement carried out by the method described in Example is 50% or less, preferably 30% or less, and further preferably 20% or less.

### EXAMPLES

Hereinafter, the present invention will be explained in more detail based on Synthetic Examples, Preparation Examples, Examples, Test Examples, etc., but the present invention is not limited to these.

### <Synthetic Example 1>

5.03 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 2.10 g of about 80% aqueous solution of methacryloylcholine chloride (available from Tokyo Chemical Industry Co., Ltd.), 2.02 g of butyl methacrylate (available from Tokyo Chemical Industry Co., Ltd.), 2.06 g of ethylene glycol dimethacrylate (available from Tokyo Chemical Industry Co., Ltd.), 48.6 g of ethanol (available from Kanto Chemical Co., Inc.) and 0.107 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 48.6 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 9.9% by mass was obtained. The resulting copolymer-containing liquid was reprecipitated with hexane, which is a poor solvent, and the precipitate was collected by filtration and dried under reduced pressure to obtain a solid copolymer.

### <Synthetic Example 2>

5.01 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 2.10 g of about 80% aqueous solution of methacryloylcholine chloride (available from Tokyo Chemical Industry Co., Ltd.), 2.31 g of butyl methacrylate (available from Tokyo Chemical Industry Co., Ltd.), 1.62 g of ethylene glycol dimethacrylate (available from Tokyo Chemical Industry Co., Ltd.), 47.9 g of ethanol (available from Kanto Chemical Co., Inc.) and 0.054 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 47.9 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 10.1% by mass was obtained. The resulting copolymer-containing liquid was reprecipitated with hexane, which is a poor solvent, and the precipitate was collected by filtration and dried under reduced pressure to obtain a solid copolymer.

### <Synthetic Example 3>

5.51 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 2.30 g of about 80% aqueous solution of methacryloylcholine chloride (available from Tokyo Chemical Industry Co., Ltd.), 1.27 g of butyl methacrylate (available from Tokyo Chemical Industry Co., Ltd.), 1.78 g of ethylene glycol dimethacrylate (available from Tokyo Chemical Industry Co., Ltd.), 47.2 g of ethanol (available from Kanto Chemical Co., Inc.) and 0.106 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 47.2 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 9.9% by mass was obtained. The resulting copolymer-containing liquid was reprecipitated with hexane, which is a poor solvent, and the precipitate was collected by filtration and dried under reduced pressure to obtain a solid copolymer.

### <Synthetic Example 4>

5.31 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 2.24 g of about 80% aqueous solution of methacryloylcholine chloride (available from Tokyo Chemical Industry Co., Ltd.), 1.63 g of butyl methacrylate (available from Tokyo Chemical Industry Co., Ltd.), 1.89 g of ethylene glycol dimethacrylate (available from Tokyo Chemical Industry Co., Ltd.), 48.2 g of ethanol (available from Kanto Chemical Co., Inc.) and 0.106 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 48.2 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 10.2% by mass was obtained. The resulting copolymer-containing liquid was reprecipitated with hexane, which is a poor solvent, and the precipitate was collected by filtration and dried under reduced pressure to obtain a solid copolymer.

### <Synthetic Example 5>

5.34 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 2.24 g of about 80% aqueous solution of methacryloylcholine chloride (available from Tokyo Chemical Industry Co., Ltd.), 1.90 g of butyl methacrylate (available from Tokyo Chemical Industry Co., Ltd.), 1.51 g of ethylene glycol dimethacrylate (available from Tokyo Chemical Industry Co., Ltd.), 47.7 g of ethanol (available from Kanto Chemical Co., Inc.) and 0.106 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 47.7 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 10.3% by mass was obtained.

### <Synthetic Example 6>

The resulting copolymer-containing liquid obtained in Synthetic Example 5 was reprecipitated with hexane, which is a poor solvent, and the precipitate was collected by filtration and dried under reduced pressure to obtain a solid copolymer.

### <Synthetic Example 7>

The resulting copolymer-containing liquid obtained in Synthetic Example 5 was reprecipitated with methyl isobutyl ketone, which is a poor solvent, and the precipitate was collected by filtration and dried under reduced pressure to obtain a solid copolymer.

### <Comparative Synthetic Example 1>

5.00 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 2.12 g of about 80% aqueous solution of methacryloylcholine chloride (available from Tokyo Chemical Industry Co., Ltd.), 1.73 g of butyl methacrylate (available from Tokyo Chemical Industry Co., Ltd.), 2.41 g of ethylene glycol dimethacrylate (available from Tokyo Chemical Industry Co., Ltd.), 48.9 g of ethanol (available from Kanto Chemical Co., Inc.) and 0.056 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 48.9 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 10.2% by mass was obtained. The resulting copolymer-containing liquid was reprecipitated with hexane, which is a poor solvent, and the precipitate was collected by filtration and dried under reduced pressure to obtain a solid copolymer.

### <Comparative Synthetic Example 2>

4.20 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 1.78 g of about 80% aqueous solution of methacryloylcholine chloride (available from Tokyo Chemical Industry Co., Ltd.), 2.89 g of butyl methacrylate (available from Tokyo Chemical Industry Co., Ltd.), 2.24 g of ethylene glycol dimethacrylate (available from Tokyo Chemical Industry Co., Ltd.), 48.9 g of ethanol (available from Kanto Chemical Co., Inc.) and 0.108 g of dimethyl-1,1'-azobis(1-cyclohexanecarboxylate) (product name: VE-073, available from FUJIFILM Wako Pure Chemical Corporation) were added and stirred uniformly to prepare a mixed liquid. On the other hand, 48.9 g of ethanol (available from Kanto Chemical Co., Inc.) was added to a four-necked flask equipped with a cooling tube, the inside of the flask was replaced with nitrogen, and the temperature was raised to the reflux temperature while stirring. While maintaining this state, the above-mentioned mixed liquid was added dropwise over 1.5 hours, and the mixture was heated and stirred for 24 hours while maintaining the above-mentioned environment after the dropwise addition. By cooling after the reaction was completed, a copolymer-containing solution having a solid content of about 10.0% by mass was obtained. The resulting copolymer-containing liquid was reprecipitated with hexane, which is a poor solvent, and the precipitate was collected by filtration and dried under reduced pressure to obtain a solid copolymer.

### <Comparative Synthetic Example 3>

3.99 g of acid phosphoxypolypropylene glycol monomethacrylate (average added mole number of propylene oxide: 5; molecular weight per a phosphate group calculated by titration: 618) (product name: PPM-5P, available from TOHO Chemical Industry Co., Ltd.), 1.67 g of about 80% aqueous solution of methacryloylcholine chloride (available from Tokyo Chemical Industry Co., Ltd.), 2.78 g of butyl methacrylate (available from Tokyo Chemical Industry Co., Ltd.), 32.5 g of ethanol (available from Kanto Chemical Co., Inc.) and 0.021 g of dimethyl-1,1'-azobis(1-cyclohexanecarboxylate) (product name: VE-073, available from FUJIFILM Wako Pure Chemical Corporation) were added and stirred uniformly to prepare a mixed liquid. The inside of the flask was replaced with nitrogen and the temperature was raised to the reflux temperature while stirring. While maintaining the above-mentioned environment for 24 hours, a copolymer-containing solution having a solid content of about 23.5% by mass was obtained.

### <Preparation Example 1>

To 0.30 g of the copolymer obtained in the above-mentioned Synthetic Example 2 were added 4.65 g of ethanol, 6.29 g of pure water and 3.00 g of 1N aqueous ammonia, and the mixture was sufficiently stirred. To the mixture was added 0.77 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.1.

### <Preparation Example 2>

To 0.50 g of the copolymer obtained in the above-mentioned Synthetic Example 3 were added 7.74 g of ethanol, 11.38 g of pure water and 5.57 g of 1N aqueous ammonia, and the mixture was sufficiently stirred. To the mixture was added 1.25 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 3>

To 0.30 g of the copolymer obtained in the above-mentioned Synthetic Example 3 were added 4.86 g of ethanol, 6.18 g of pure water and 3.01 g of 1N aqueous ammonia, and the mixture was sufficiently stirred. To the mixture was added 1.50 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 4>

To 0.30 g of the copolymer obtained in the above-mentioned Synthetic Example 4 were added 4.75 g of ethanol, 6.24 g of pure water and 3.01 g of 1N aqueous ammonia, and the mixture was sufficiently stirred. To the mixture was added 1.12 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 5>

To 0.10 g of the copolymer obtained in the above-mentioned Synthetic Example 4 were added 3.31 g of ethanol, 6.10 g of pure water and 1.13 g of 1N aqueous ammonia, and the mixture was sufficiently stirred. To the mixture was added 0.25 g of a solution obtained by diluting Carbodilite V-02-L2 (available from Nisshinbo Chemical Inc., solid content: about 40% by mass) 10-fold with pure water and the mixture was sufficiently stirred to prepare a composition for forming a coating film. The pH was 10.0.

### <Preparation Example 6>

To 0.30 g of the copolymer obtained in the above-mentioned Synthetic Example 6 were added 4.76 g of ethanol, 6.31 g of pure water and 3.01 g of 1N aqueous ammonia, and the mixture was sufficiently stirred. To the mixture was added 1.13 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 7>

To 0.10 g of the copolymer obtained in the above-mentioned Synthetic Example 6 were added 3.32 g of ethanol, 6.25 g of pure water and 1.13 g of 1N aqueous ammonia, and the mixture was sufficiently stirred. To the mixture was added 0.38 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 8>

To 0.10 g of the copolymer obtained in the above-mentioned Synthetic Example 6 were added 3.08 g of ethanol, 5.97 g of pure water and 1.16 g of 1N aqueous ammonia, and the mixture was sufficiently stirred. To the mixture was added 0.13 g of a solution obtained by diluting Carbodilite V-02-L2 (available from Nisshinbo Chemical Inc., solid content: about 40% by mass) 10-fold with pure water and the mixture was sufficiently stirred to prepare a composition for forming a coating film. The pH was 10.0.

### <Preparation Example 9>

To 5.00 g of the copolymer-containing liquid obtained in the above-mentioned Synthetic Example 5 were added 3.68 g of ethanol, 12.12 g of pure water and 5.16 g of 1N aqueous ammonia, and the mixture was sufficiently stirred. To the mixture was added 1.94 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 10>

To 0.50 g of the copolymer obtained in the above-mentioned Synthetic Example 7 were added 7.91 g of ethanol, 11.74 g of pure water and 5.01 g of 1 N aqueous ammonia, and the mixture was sufficiently stirred. To the mixture was added 1.88 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 11>

To 0.90 g of the copolymer obtained in the above-mentioned Synthetic Example 7 were added 13.91 g of ethanol, 21.44 g of pure water and 9.01 g of 1N aqueous ammonia, and the mixture was sufficiently stirred. To the mixture was added 2.25 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 12>

To 0.50 g of the copolymer obtained in the above-mentioned Synthetic Example 7 were added 7.55 g of ethanol, 12.08 g of pure water and 5.00 g of 1N aqueous ammonia, and the mixture was sufficiently stirred. To the mixture was added 0.63 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 13>

To 5.00 g of the copolymer-containing liquid obtained in the above-mentioned Synthetic Example 5 were added 3.11 g of ethanol, 11.55 g of pure water and 5.14 g of 1N aqueous ammonia, and the mixture was sufficiently stirred. To the mixture was added 1.24 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Preparation Example 14>

To 5.00 g of the copolymer-containing liquid obtained in the above-mentioned Synthetic Example 5 were added 2.93 g of ethanol, 11.76 g of pure water and 5.08 g of 1N aqueous ammonia, and the mixture was sufficiently stirred. To the mixture was added 0.62 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Comparative Preparation Example 1>

To 0.80 g of the copolymer obtained in the above-mentioned Comparative Synthetic Example 1 were added 27.45 g of ethanol and 10.58 g of pure water, and the mixture was sufficiently stirred to prepare a composition for forming a coating film. The pH was 2.9.

### <Comparative Preparation Example 2>

To 0.80 g of the copolymer-containing liquid obtained in the above-mentioned Comparative Synthetic Example 1 were added 12.36 g of ethanol, 15.77 g of pure water and 9.00 g of 1N aqueous ammonia, and the mixture was sufficiently stirred. To the mixture was added 2.00 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.1.

### <Comparative Preparation Example 3>

To 0.25 g of the copolymer obtained in the above-mentioned Comparative Synthetic Example 2 were added 8.58 g of ethanol and 3.68 g of pure water, and the mixture was sufficiently stirred to prepare a composition for forming a coating film. The pH was 2.9.

### <Comparative Preparation Example 4>

To 0.80 g of the copolymer obtained in the above-mentioned Synthetic Example 1 were added 27.51 g of ethanol and 11.76 g of pure water, and the mixture was sufficiently stirred to prepare a composition for forming a coating film. The pH was 2.8.

### <Comparative Preparation Example 5>

To 0.25 g of the copolymer obtained in the above-mentioned Synthetic Example 2 were added 8.59 g of ethanol and 3.70 g of pure water, and the mixture was sufficiently stirred to prepare a composition for forming a coating film. The pH was 2.9.

### <Comparative Preparation Example 6>

To 0.26 g of the copolymer obtained in the above-mentioned Synthetic Example 3 were added 8.60 g of ethanol and 3.69 g of pure water, and the mixture was sufficiently stirred to prepare a composition for forming a coating film. The pH was 2.8.

### <Comparative Preparation Example 7>

To 3.02 g of the copolymer-containing liquid obtained in the above-mentioned Comparative Synthetic Example 3 were added 8.87 g of ethanol, 14.47 g of pure water and 7.04 g of 1N aqueous ammonia, and the mixture was sufficiently stirred. To the mixture was added 2.66 g of a solution obtained by diluting Carbodilite V-02 (available from Nisshinbo Chemical Inc., solid content: about 40% by mass) 10-fold with pure water and the mixture was sufficiently stirred to prepare a composition for forming a coating film. The pH was 10.0.

### <Test Example 1: Evaluation of dissolution of coating film>

The compositions for forming a coating film obtained in Preparation Examples 1 to 14 and Comparative Preparation Examples 1 to 7 were each spin-coated on an HMDS-treated silicon wafer at 1500 rpm/60 sec, and dried under the drying conditions shown in Table 1. Subsequently, washing with pure water or a mixed solution of pure water and ethanol was carried out and then drying was carried out at 50°C for 1 hour to obtain a coating film on the HMDS-treated silicon wafer. The film thickness was measured with a spectroscopic ellipsometer, and the film thickness at this time was defined as the initial film thickness. As a dissolution test of the coating film, the film was immersed in PBS for 24 hours, then washed with pure water and dried at 50° C for 1 hour, and the film thickness was measured with a spectroscopic ellipsometer. The initial film thickness and the film thickness after immersion in PBS were compared, and the ratio of residual film after immersion in PBS when the initial film thickness was taken as 100% was used as an index of dissolution. The results are shown in Table 1.

### <Test Example 2: Evaluation of protein adsorption>

### (Preparation of coating plate)

The compositions for forming a coating film obtained in Preparation Examples 1 to 14 and Comparative Preparation Examples 1 to 7 were each added to a 96-well plate (manufactured by Corning, Inc., #3363, volume 0.32 mL, made of polypropylene) so as to be 150 µL/well per each 5 wells. After allowing to stand at room temperature for 1 hour, the liquid was drained and the well was dried under the drying conditions shown in Table 1 using an oven. Thereafter, each well was washed with 200 µL of pure water or a mixed solution of pure water and ethanol each three times, and dried using an oven at 50°C for 1 hour to prepare a coating plate. As a negative control, wells of an uncoated 96-well plate (manufactured by Corning, Inc., #3363, volume 0.32 mL, made of polypropylene) were used.

### (Preparation of IgG-HRP diluted solution)

A goat anti-mouse IgG antibody-HRP conjugate (manufactured by Southern Biotechnology Associates) was diluted with PBS so as to have a concentration of 1 mg/g to prepare an IgG-HRP diluted solution.

### (Evaluation of protein adsorption)

100 µL/well of IgG-HRP diluted solution was added to each well of the plate prepared as mentioned above and to the negative control, and allowed to stand at room temperature for 30 minutes. Thereafter, the IgG-HRP diluted solutions were then drained and each well washed with 200 µL of PBS each three times. TMB solution (manufactured by sera care, SureBlue) was added at 100 µL/well, and after 1 minute, TMB STOP solution (manufactured by sera care) was added at 100 µL/well. Using a microplate reader (manufactured by TECAN, infinite M200PRO), absorbances at 450 nm and 650 nm were measured. A value obtained by subtracting the absorbance at 650 nm from the absorbance at 450 nm was calculated to obtain an average absorbance of 5 wells for each composition for forming a coating film. The results are shown in Table 1.

### <Test Example 3: Evaluation of antibody aggregation>

### (Preparation of coating tube)

The compositions for forming a coating film obtained in Preparation Examples 1 to 14 and Comparative Preparation Examples 1 to 7 were each charged in microtubes made of polypropylene (PP) (manufactured by NIPPON Genetics Co., Ltd., #11510) in an amount of 1.5 mL and allowed to stand at 25°C for 0.5 hour. After removing the composition for forming a coating film from the tube, the tube was dried under the drying conditions shown in Table 1. Thereafter, the tube was sufficiently washed with pure water or a mixed solution of pure water and ethanol to obtain a coating tube having a coating film formed thereon. As a negative control, an uncoated microtube made of polypropylene (PP) (manufactured by NIPPON Genetics Co., Ltd., #11510) was used.

### (Evaluation of antibody aggregation)

After purifying the rituximab solution and adjusting the concentration to 1.0 mg/mL, it was subjected to filtration and sterilization treatment by a 0.22 µm filter under a sterile environment. The prepared rituximab solution was filled in the coating tube obtained as mentioned above with each 0.5 mL, and the tube was set in a microtube stirring shaker, and shaken at 22±3°C and 2500 rpm for 24 hours. The solution was replaced from the tube after stirring and shaking to a transparent vial, and the appearance of cloudiness was visually evaluated according to the following evaluation standard to confirm the effect of suppressing formation of aggregation. The results are shown in Table 1.

### [Evaluation standard]

∘: Colorless and transparent without turbidity
△: Slightly turbid
×: Turbid

**[Table 1]**

| | Coating agent | Drying conditions | Washing solvent Pure water! ethanol | Evaluation of dissolution | | | Evaluation of protein adsorption | Evaluation of antibody aggregation |
|---|---|---|---|---|---|---|---|---|
| | | | | Initial film thickness [nm] | Film thickness after dipping [nm] | Film remaining ratio | Absorbance (450nm -650nm) | |
| Example1 | Preparation Example 1 | 50°C/3h | 10wt/0wt | 49.0 | 46.7 | 95.3% | 0.019 | ○ |
| Example 2 | Preparation Example 2 | 50°C/3h | 10wt/0wt | 31.5 | 28.3 | 89.8% | 0.030 | ○ |
| Example 3 | Preparation Example 3 | 50°C/3h | 10wt/0wt | 35.0 | 32.1 | 91.9% | 0.034 | Δ |
| Example 4 | Preparation Example 4 | 50°C/3h | 10wt/0wt | 32.7 | 30.8 | 94.3% | 0.017 | ○ |
| Example 5 | Preparation Example 5 | 50°C/3h | 10wt/0wt | 25.3 | 23.4 | 92.5% | 0.013 | ○ |
| Example 6 | Preparation Example 6 | 50°C/3h | 10wt/0wt | 29.9 | 27.6 | 92.3% | 0.015 | ○ |
| Example 7 | Preparation Example 7 | 50°C/3h | 10wt/0wt | 27.1 | 25.0 | 92.4% | 0.017 | ○ |
| Example 8 | Preparation Example 8 | 50°C/3h | 10wt/0wt | 28.8 | 26.4 | 91.6% | 0.010 | ○ |
| Example 9 | Preparation Example 9 | 50°C/3h | 10wt/0wt | 40.5 | 37.3 | 92.0% | 0.039 | ○ |
| Example 10 | Preparation Example 9 | 50°C/3h | 7wt/3wt | 35.0 | 33.6 | 95.9% | 0.027 | ○ |
| Example 11 | Preparation Example 10 | 50°C/3h | 10wt/0wt | 40.4 | 39.1 | 96.8% | 0.039 | ○ |
| Example 12 | Preparation Example 10 | 50°C/3h | 7wt/3wt | 42.1 | 41.6 | 98.9% | 0.026 | ○ |
| Example 13 | Preparation Example 11 | 50°C/3h | 10wt/0wt | 47.9 | 45.7 | 95.4% | 0.021 | ○ |
| Example 14 | Preparation Example 11 | 50°C/3h | 7wt/3wt | 37.2 | 37.0 | 99.5% | 0.032 | ○ |
| Example 15 | Preparation Example 12 | 50°C/3h | 10wt/0wt | 43.2 | 37.9 | 87.8% | 0.020 | ○ |
| Example 16 | Preparation Example 12 | 50°C/3h | 7wt/3wt | 39.1 | 37.6 | 96.1% | 0.024 | ○ |
| Example 17 | Preparation Example 13 | 50°C/3h | 3wt/7wt | 40.4 | 40.2 | 99.3% | 0.017 | ○ |
| Example 18 | Preparation Example 14 | 50°C/3h | 7wt/3wt | 52.6 | 51.2 | 97.3% | 0.010 | ○ |
| Example 19 | Preparation Example 14 | 50°C/3h | 3wt/7wt | 35.0 | 32.2 | 91.9% | 0.013 | ○ |
| Example 20 | Preparation Example 14 | 90°C/24h | 3wt/7wt | 38.3 | 37.1 | 96.9% | 0.014 | ○ |
| Comparative Example 1 | None | - | - | - | - | - | 0.728 | × |
| Comparative Example 2 | Comparative Preparation Example 1 | 50°C/3h | 10wt/0wt | 43.8 | 39.7 | 90.7% | 0.028 | × |
| Comparative Example 3 | Comparative Preparation Example 2 | 50°C/3h | 10wt/0wt | 20.9 | 20.3 | 96.9% | 0.019 | × |
| Comparative Example 4 | Comparative Preparation Example 3 | 50°C/3h | 10wt/0wt | 107.0 | 105.7 | 98.9% | 0.032 | × |
| Comparative Example 5 | Comparative Preparation Example 4 | 50°C/3h | 10wt/0wt | 125.6 | 111.7 | 88.9% | 0.022 | Δ |
| Comparative Example 6 | Comparative Preparation Example 5 | 50°C/3h | 10wt/0wt | 111.3 | 99.4 | 89.3% | 0.014 | ○ |
| Comparative Example 7 | Comparative Preparation Example 6 | 50°C/3h | 10wt/0wt | 97.0 | 45.2 | 46.6% | 0.032 | ○ |
| Comparative Example 8 | Comparative Preparation Example 7 | 50°C/3h | 10wt/0wt | 25.1 | 22.6 | 90.0% | 0.002 | ○ |

As shown in Comparative Example 1, in the case of no coating film, the solution after the evaluation of antibody aggregation was turbid and formation of aggregates was confirmed. Also, in Comparative Examples 2 to 7, in the evaluation of protein adsorption, the absorbance was significantly reduced as compared to Comparative Example 1 having no coating film, and suppression of protein adsorption was confirmed. However, in the evaluation of antibody aggregation of Comparative Examples 2 to 4 using the coating agents that does not contain the polymer according to the present invention, it was confirmed the state that the solution is turbid and it could be found that no suppressing effect of antibody aggregation was observed. Further, in Comparative Examples 5 to 7 using the coating agents that contain the polymer according to the present invention but does not contain the polycarbodiimide as a crosslinking agent, the suppressing effect of antibody aggregation was observed but as compared to the coating agent of the present invention containing the same polymer, the film remaining ratio is low and tendency of dissolution of the coating film was observed (for example, see Example 1 and Comparative Example 6, and Example 2 or 3 and Comparative Example 7). To the contrary, in Examples 1 to 20, it could be confirmed that these were the coating films well balanced in desired characteristics that the film remaining ratio is high so that dissolution of the coating film is suppressed, and also excellent in suppressing ability of protein adsorption and suppressing ability of antibody aggregation.

### UTILIZABILITY IN INDUSTRY

According to the present invention, it can be provided a composition for forming a coating film having an ability to suppress adhesion of biological substances and an ability to suppress aggregation of antibody drugs, a coating film which is a cured product thereof, and a process for producing the same, and a cured product and a process for producing the same. Further, the coating film of the present invention is practically superior in that dissolution of the components of the coating film into pharmaceuticals is suppressed.

## Claims

1. A composition for forming a coating film which comprises
(1) a polymer of a monomer mixture which contains an anionic monomer represented by the following formula (A): 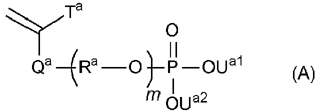 wherein
T^{a}, U^{a1} and U^{a2} each independently represent a hydrogen atom or a linear or branched alkyl group having 1 to 5 carbon atoms;
Q^{a} represents a single bond, an ester bond or an amide bond;
R^{a} represents a linear or branched alkylene group having 1 to 10 carbon atoms which may be substituted by a halogen atom(s); and
m represents an integer of 1 to 10,
a cationic monomer represented by the following formula (B): 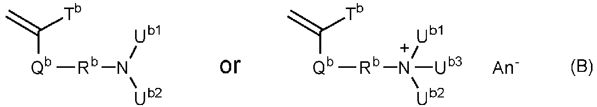 wherein
T^{b}, U^{b1}, U^{b2} and U^{b3} each independently represent a hydrogen atom or a linear or branched alkyl group having 1 to 5 carbon atoms;
Q^{b} represents a single bond, an ester bond or an amide bond;
R^{b} represents a linear or branched alkylene group having 1 to 10 carbon atoms which may be substituted by a halogen atom(s); and
An⁻ represents an anion selected from the group consisting of a halide ion, an inorganic acid ion, a hydroxide ion and an isothiocyanate ion,
a hydrophobic monomer represented by the following formula (C): 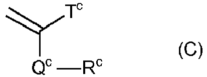 wherein
T^{c} represents a hydrogen atom or a linear or branched alkyl group having 1 to 5 carbon atoms;
Q^{c} represents a single bond, an ether bond or an ester bond;
R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms, where the aryl portion may be substituted by a linear or branched alkyl group having 1 to 5 carbon atoms which may be substituted by a halogen atom(s), and a bifunctional monomer represented by the following formula (D): 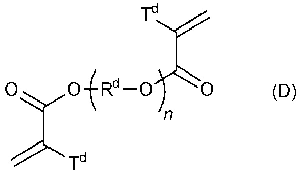 wherein
T^{d} represents a hydrogen atom or a linear or branched alkyl group having 1 to 6 carbon atoms;
R^{d} represents a linear or branched alkylene group having 1 to 10 carbon atoms which may be substituted by a halogen atom(s); and
n represents an integer of 1 to 10,
where a ratio of a total of the anionic monomer represented by the above-mentioned formula (A) and the cationic monomer represented by the above-mentioned formula (B) based on total monomers contained in the monomer mixture is 40 mol% or more, and
(2) a polycarbodiimide containing a structure represented by the following formula (E):
-N=C=N- (E)

2. The composition for forming a coating film according to Claim 1, wherein a ratio of the bifunctional monomer represented by the formula (D) based on the total monomers contained in the monomer mixture is less than 30 mol%.

3. The composition for forming a coating film according to Claim 1 or 2, wherein the polycarbodiimide contains a hydrophilic group(s).

4. The composition for forming a coating film according to Claim 3, wherein the hydrophilic group is represented by the following formula (F):
R¹-(O-CH²-CH₂)*ₒ*- (F)

5. A coating film which comprises a cured product of an applied film of the composition for forming a coating film according to any one of Claims 1 to 4.

6. The coating film according to Claim 5, which has an ability to suppress adhesion of biological substances.

7. A process for producing a coating film which comprises a step of applying a composition for forming a coating film according to any one of Claims 1 to 4 onto the substrate to form an applied film, and a step of drying the applied film to form a cured product.

8. The process for producing a coating film according to Claim 7, which further comprises a step of washing the cured product obtained after the drying step with a water-containing alcohol solvent.

9. A cured product of a polymer of a monomer mixture which comprises
an anionic monomer represented by the following formula (A): 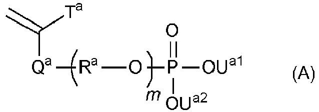 wherein
T^{a}, U^{a1} and U^{a2} each independently represent a hydrogen atom or a linear or branched alkyl group having 1 to 5 carbon atoms;
Q^{a} represents a single bond, an ester bond or an amide bond;
R^{a} represents a linear or branched alkylene group having 1 to 10 carbon atoms which may be substituted by a halogen atom(s); and
m represents an integer of 1 to 10,
a cationic monomer represented by the following formula (B): 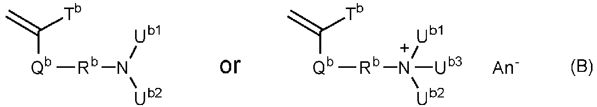 wherein
T^{b}, U^{b1}, U^{b2} and U^{b3} each independently represent a hydrogen atom or a linear or branched alkyl group having 1 to 5 carbon atoms;
Q^{b} represents a single bond, an ester bond or an amide bond;
R^{b} represents a linear or branched alkylene group having 1 to 10 carbon atoms which may be substituted by a halogen atom(s); and
An⁻ represents an anion selected from the group consisting of a halide ion, an inorganic acid ion, a hydroxide ion and an isothiocyanate ion,
a hydrophobic monomer represented by the following formula (C): 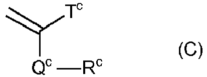 wherein
T^{c} represents a hydrogen atom or a linear or branched alkyl group having 1 to 5 carbon atoms;
Q^{c} represents a single bond, an ether bond or an ester bond;
R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms, where the aryl portion may be substituted by a linear or branched alkyl group having 1 to 5 carbon atoms which may be substituted by a halogen atom(s), and a bifunctional monomer represented by the following formula (D): 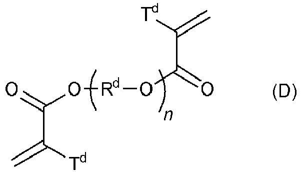 wherein
T^{d} represents a hydrogen atom or a linear or branched alkyl group having 1 to 6 carbon atoms;
R^{d} represents a linear or branched alkylene group having 1 to 10 carbon atoms which may be substituted by a halogen atom(s); and
n represents an integer of 1 to 10,
where a ratio of a total of the anionic monomer represented by the above-mentioned formula (A) and the cationic monomer represented by the above-mentioned formula (B) based on total monomers contained in the monomer mixture is 40 mol% or more.

10. The cured product according to Claim 9, which contains a pyrophosphate structure.

11. A process for producing a cured product, which comprises
(i) a step of polymerizing a monomer mixture containing an anionic monomer represented by the following formula (A): 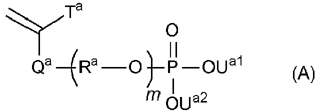 wherein
T^{a}, U^{a1} and U^{a2} each independently represent a hydrogen atom or a linear or branched alkyl group having 1 to 5 carbon atoms;
Q^{a} represents a single bond, an ester bond or an amide bond;
R^{a} represents a linear or branched alkylene group having 1 to 10 carbon atoms which may be substituted by a halogen atom(s); and
m represents an integer of 1 to 10,
a cationic monomer represented by the following formula (B): 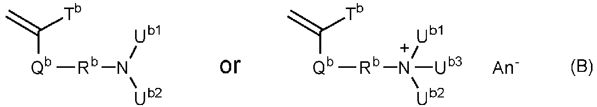 wherein
T^{b}, U^{b1}, U^{b2} and U^{b3} each independently represent a hydrogen atom or a linear or branched alkyl group having 1 to 5 carbon atoms;
Q^{b} represents a single bond, an ester bond or an amide bond;
R^{b} represents a linear or branched alkylene group having 1 to 10 carbon atoms which may be substituted by a halogen atom(s); and
An⁻ represents an anion selected from the group consisting of a halide ion, an inorganic acid ion, a hydroxide ion and an isothiocyanate ion,
a hydrophobic monomer represented by the following formula (C): 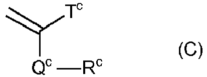 wherein
T^{c} represents a hydrogen atom or a linear or branched alkyl group having 1 to 5 carbon atoms;
Q^{c} represents a single bond, an ether bond or an ester bond;
R^{c} represents a linear or branched alkyl group having 1 to 18 carbon atoms, a cyclic hydrocarbon group having 3 to 10 carbon atoms, an aryl group having 6 to 10 carbon atoms, an aralkyl group having 7 to 14 carbon atoms or an aryloxyalkyl group having 7 to 14 carbon atoms, where the aryl portion may be substituted by a linear or branched alkyl group having 1 to 5 carbon atoms which may be substituted by a halogen atom(s), and a bifunctional monomer represented by the following formula (D): 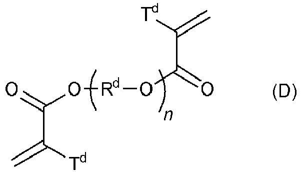 wherein
T^{d} represents a hydrogen atom or a linear or branched alkyl group having 1 to 6 carbon atoms;
R^{d} represents a linear or branched alkylene group having 1 to 10 carbon atoms which may be substituted by a halogen atom(s); and
n represents an integer of 1 to 10,
where a ratio of a total of the anionic monomer represented by the formula (A) and the cationic monomer represented by the formula (B) based on total monomers contained in the monomer mixture is 40 mol% or more to obtain a copolymer, and
(ii) a step of reacting the copolymer with a polycarbodiimide containing the structure represented by the following formula (E):
-N=C=N- (E)
to obtain a cured product.

12. The coating film according to Claim 5, which has an ability to suppress aggregation of antibody.

13. A storage container of an antibody drug, which comprises having the coating film according to Claim 12 onto at least part of the surface thereof.
